(19)

Europäisches Patentamt
European Patent Office
Office européen des brevets

(11) **EP 4 730 345 A1**

(12) **EUROPEAN PATENT APPLICATION**
published in accordance with Art. 153(4) EPC

(43) Date of publication:
**22.04.2026 Bulletin 2026/17**

(21) Application number: **24823389.2**

(22) Date of filing: **11.06.2024**

(51) International Patent Classification (IPC):
**G16C 20/30** (2019.01)          **C12Q 1/02** (2006.01)
**G01N 33/50** (2006.01)

(52) Cooperative Patent Classification (CPC):
**C12Q 1/02; G01N 33/50; G16C 20/30**

(86) International application number:
**PCT/JP2024/021249**

(87) International publication number:
**WO 2024/257774 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.06.2023 JP 2023098741**

(71) Applicant: **FUJIFILM Corporation**
**Tokyo 106-8620 (JP)**

(72) Inventors:
• **TATESHITA, Masakazu**
  **Ashigarakami-gun, Kanagawa 258-8577 (JP)**
• **SUGIYAMA, Satoshi**
  **Ashigarakami-gun, Kanagawa 258-8577 (JP)**
• **HIKIDA, Yasushi**
  **Ashigarakami-gun, Kanagawa 258-8577 (JP)**
• **SHIRAISHI, Yasushi**
  **Ashigarakami-gun, Kanagawa 258-8577 (JP)**
• **MIYOSHI, Hayato**
  **Ashigarakami-gun, Kanagawa 258-8577 (JP)**

(74) Representative: **HGF**
**HGF Limited**
**4th Floor, 1 City Square**
**Leeds LS1 2ES (GB)**

(54) **DRUG DISCOVERY ASSISTANCE DEVICE, METHOD FOR OPERATING DRUG DISCOVERY ASSISTANCE DEVICE, AND PROGRAM FOR OPERATING DRUG DISCOVERY ASSISTANCE DEVICE**

(57) A drug discovery support apparatus includes a processor, and the processor is configured to: acquire a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed; derive an index value indicating a dose-response relationship of the candidate substance for each of the ion channels, based on the first prediction value for each added amount; and present, to a user, estimated reference information that corresponds to the index value and is referred to in order to estimate a mechanism of action of the candidate substance.

FIG. 4

EP 4 730 345 A1

**Description**

BACKGROUND OF THE INVENTION

1. Field of the Invention

The technology of the present disclosure relates to a drug discovery support apparatus, a method for operating a drug discovery support apparatus, and a program for operating a drug discovery support apparatus.

2. Description of the Related Art

As a test for evaluating the safety of a candidate substance for a drug, a test for evaluating whether or not the candidate substance has cardiotoxicity is required in the non-clinical safety pharmacology test guideline S7B of the International Council for Harmonisation of Technical Requirements for Pharmaceuticals for Human Use (ICH). Among the cardiotoxicities, a fatal arrhythmia called Torsades de Pointes (TdP) is regarded as the most important toxicity. The cause of the fatal arrhythmia is believed to be a phenomenon in which a QT interval of an electrocardiogram is prolonged, that is, QT prolongation.

In the test according to the related art based on the ICH S7B guideline, the inhibitory action of candidate substances on a human Ether-a-go-go Related Gene (hERG) channel, which is one of ion channels expressed in the myocardial cells, is examined, and the QT interval of an electrocardiogram is evaluated using non-human animals. Then, a candidate substance with a high probability of causing the fatal arrhythmias is extracted. However, a substance having a strong inhibitory action on the hERG channel does not necessarily cause QT prolongation. Therefore, in order to improve the accuracy of the evaluation, it is necessary to evaluate the influence on other ion channels such as a Ca channel and a Na channel.

By the way, myocardial cells (hereinafter, referred to as iPS myocardial cells) derived from human induced Pluripotent Stem (iPS) cells also have ion channels expressed in human cardiac tissues, such as the Na channel and the Ca channel, in addition to the hERG channel. For this reason, in the related art, the usefulness of the iPS myocardial cells has been verified as a tool that can evaluate whether or not QT prolongation occurs in a candidate substance for a drug. In this regard, a method that measures the electrical activity of iPS myocardial cells as an electrocardiogram-like waveform using a planar microelectrode array (MEA) and evaluates whether or not QT prolongation occurs has been shown to be useful by performing a large-scale multi-center verification test and has been included in the follow-up test of the ICH S7B guideline. In addition, specifically, the electrocardiogram-like waveform is a fluctuation waveform of the extracellular potential of the iPS myocardial cell.

In the method of measuring the electrical activity of the iPS myocardial cells using MEA, it is possible to know whether or not QT prolongation occurs. However, in a case where QT prolongation occurs, it is not possible to know the mechanism of action of the candidate substance such as in which of the ion channels the flow of ions is inhibited or activated by the candidate substance, resulting in QT prolongation. Therefore, K. H. Jaeger, et al. "Identifying Drug Response by Combining Measurements of the Membrane Potential, the Cytosolic Calcium Concentration, and the Extracellular Potential in Microphysiological Systems" Frontiers in Pharmacology 08 February 2021. (hereinafter, referred to as Non-Patent Document 1) and Fabien Raphel, et al. "A greedy classifier optimization strategy to assess ion channel blocking activity and pro-arrhythmia in hiPSC-cardiomyocytes" PLOS Computational Biology September 25, 2020. (hereinafter, referred to as Non-Patent Document 2) disclose a technique that contributes to the estimation of the mechanism of action of a candidate substance.

In Non-Patent Document 1, the height of a first peak and the conduction velocity of a fluctuation waveform are derived as feature amounts from the fluctuation waveform of the extracellular potential of the iPS myocardial cell measured by MEA, and the derived feature amounts are input to a prediction model such that a prediction value of the degree of inhibition of the candidate substance on the Na channel (a numerical value indicating the degree to which the flow of Na ions in the Na channel is inhibited by the candidate substance) is output from the prediction model. In addition, the degree of inhibition of the candidate substance on the hERG channel (a numerical value indicating the degree to which the flow of K ions in the hERG channel is inhibited by the candidate substance) is obtained by measuring the intracellular potential of the iPS myocardial cell. Further, the degree of inhibition of the candidate substance on the Ca channel (a numerical value indicating the degree to which the flow of Ca ions in the Ca channel is inhibited by the candidate substance) is obtained by measuring calcium concentration.

In Non-Patent Document 2, a simulation model is used to generate a fluctuation waveform of the intracellular potential of the iPS myocardial cell, and the generated fluctuation waveform of the intracellular potential of the iPS myocardial cell is converted into a fluctuation waveform of the extracellular potential of the iPS myocardial cell by MEA. Then, an algorithm that predicts the degrees of inhibition of the candidate substance on the hERG channel, the Na channel, and the Ca channel based on the feature amounts derived from the fluctuation waveform of the extracellular potential of the iPS myocardial cell is proposed.

SUMMARY OF THE INVENTION

It is considered that an index value indicating a dose-response relationship of the candidate substance, such as the median inhibitory concentration (IC50), is very useful for estimating the mechanism of action of the candidate substance. However, the techniques disclosed in Non-Patent Documents 1 and 2 do not derive the index value. Therefore, there is a concern that it may take a lot of time and effort to estimate the mechanism of action of the candidate substance.

An embodiment according to the technology of the present disclosure provides a drug discovery support apparatus, a method for operating a drug discovery support apparatus, and a program for operating a drug discovery support apparatus that can easily estimate a mechanism of action of a candidate substance.

According to an aspect of the present disclosure, there is provided a drug discovery support apparatus comprising a processor, in which the processor is configured to: acquire a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed; derive an index value indicating a dose-response relationship of the candidate substance for each of the ion channels, based on the first prediction value for each added amount; and present, to a user, estimated reference information that corresponds to the index value and is referred to in order to estimate a mechanism of action of the candidate substance.

Preferably, the processor is configured to: search for a dose-response curve suitable for the first prediction value for each added amount; and derive the index value from the searched dose-response curve.

Preferably, the first prediction value is not only a prediction value of the degree of inhibition but also a prediction value of a degree of activity indicating a degree to which the flow of the ions in the ion channel is activated by the candidate substance, the dose-response curves are of two types: a first dose-response curve in a case where the flow of the ions in the ion channel is inhibited by the candidate substance; and a second dose-response curve in a case where the flow of the ions in the ion channel is activated by the candidate substance, and the processor is configured to derive the index value from one of the first dose-response curve and the second dose-response curve which is more suitable for the first prediction value for each added amount.

Preferably, the processor is configured to present the searched dose-response curve as the estimated reference information to the user.

Preferably, a curve used to search for the dose-response curve is a logistic curve.

Preferably, the first prediction value is obtained by inputting a feature amount derived from the first fluctuation waveform to the prediction model.

Preferably, the processor is configured to: acquire the first fluctuation waveform; derive the feature amount from the first fluctuation waveform; and input the feature amount to the prediction model such that the first prediction value is output from the prediction model.

Preferably, the processor is configured to perform a noise reduction process on the first fluctuation waveform prior to the derivation of the feature amount.

Preferably, the first fluctuation waveform has periodicity corresponding to beating of the iPS myocardial cell, and the processor is configured to perform, as the noise reduction process, a process of adding and averaging a plurality of periodic portions of the first fluctuation waveform.

Preferably, the first fluctuation waveform is measured for one iPS myocardial cell by a plurality of electrodes, and the processor is configured to select one of a plurality of the first fluctuation waveforms measured by the plurality of electrodes as the first fluctuation waveform from which the feature amount is derived, according to a preset condition.

Preferably, the feature amount includes a conduction velocity of the first fluctuation waveform.

Preferably, the feature amount is standardized by a reference feature amount derived from a reference first fluctuation waveform measured in a case where the candidate substance is not added to the iPS myocardial cell.

Preferably, in a case where there is an experimental value of the index value indicating the dose-response relationship of the candidate substance, the first prediction value is obtained by inputting the experimental value to the prediction model in addition to the feature amount.

Preferably, the prediction model is a model that has been trained in two distinct cases: a case where the experimental value is input; and a case where the experimental value is not input.

Preferably, the first prediction value is obtained by inputting a second prediction value of the index value indicating the dose-response relationship of the candidate substance, which has been derived based on structural information of the candidate substance, to the prediction model in addition to the feature amount.

Preferably, the prediction model is a model that has been trained using learning data including simulation data.

Preferably, the simulation data is generated using a first simulation model that reproduces a second fluctuation waveform, which is a fluctuation waveform of an intracellular potential of the iPS myocardial cell, and a second simulation model that converts the second fluctuation waveform into the first fluctuation waveform.

According to another aspect of the present disclosure, there is provided a method for operating a drug discovery support apparatus, the method comprising: acquiring a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed; deriving an index value indicating a dose-response relationship of the candidate substance for each of the ion channels, based on the first prediction value for each added amount; and presenting, to a user, estimated reference information that corresponds to the index value and is referred to in order to estimate a mechanism of action of the candidate substance.

According to still another aspect of the present disclosure, there is provided a program for operating a drug discovery support apparatus, the program causing a computer to execute a process comprising: acquiring a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed; deriving an index value indicating a dose-response relationship of the candidate substance for each of the ion channels, based on the first prediction value for each added amount; and presenting, to a user, estimated reference information that corresponds to the index value and is referred to in order to estimate a mechanism of action of the candidate substance.

According to the technology of the present disclosure, it is possible to provide a drug discovery support apparatus, a method for operating a drug discovery support apparatus, and a program for operating a drug discovery support apparatus that can easily estimate a mechanism of action of a candidate substance.

BRIEF DESCRIPTION OF THE DRAWINGS

FIG. 1 is a diagram showing a drug discovery support server, a user terminal, and a well plate.

FIG. 2 is a table showing an amount of candidate substance for a drug added to each well.

FIG. 3 is a block diagram showing computers constituting the drug discovery support server and the user terminal.

FIG. 4 is a block diagram showing processing units of a CPU of the drug discovery support server.

FIG. 5 is a diagram showing a process of a preprocessing unit.

FIG. 6 is a diagram showing a noise reduction process.

FIG. 7 is a diagram showing a selection process.

FIG. 8 is a diagram showing the selection process.

FIG. 9 is a diagram showing a process of a feature amount derivation unit.

FIG. 10 is a diagram showing an aspect in which feature amounts are standardized by reference feature amounts.

FIG. 11 is a diagram showing feature amount information.

FIG. 12 is a diagram showing a prediction model group.

FIG. 13 is a diagram showing a process of a prediction unit that inputs the feature amount to each prediction model such that a first prediction value is output from each prediction model.

FIG. 14 is a diagram showing prediction result information.

FIG. 15 is a diagram showing a process in a training phase of an hERG channel prediction model.

FIG. 16 is a diagram showing a dose-response curve group.

FIG. 17 is a graph showing a first dose-response curve.

FIG. 18 is a graph showing a second dose-response curve.

FIG. 19 is a flowchart showing a processing procedure of a search unit.

FIG. 20 is a diagram showing an aspect in which the first prediction value is derived for each ion channel and for each added amount.

FIG. 21 is a diagram showing an aspect in which a squared error of a searched second dose-response curve is smaller than a squared error of a searched first dose-response curve and a median effective concentration is derived from the searched second dose-response curve.

FIG. 22 is a block diagram showing processing units of a CPU of the user terminal.

FIG. 23 is a diagram showing a waveform input screen.

FIG. 24 is a diagram showing an evaluation result display screen.

FIG. 25 is a flowchart showing a processing procedure of the drug discovery support server.

FIG. 26 is a diagram showing a second embodiment in which, in addition to the feature amount, an experimental value of an index value indicating a dose-response relationship of the candidate substance is input to the prediction model.

FIG. 27 is a diagram showing a process in a training phase of an hERG channel prediction model according to the second embodiment.

FIG. 28 is a diagram showing a third embodiment in which, in addition to the feature amount, a second prediction value of the index value indicating the dose-response relationship of the candidate substance, which has been derived based on structural information of the candidate substance, is input to the prediction model.

FIG. 29 is a diagram showing a fourth embodiment in which simulation data is included in learning data of the prediction model.

FIG. 30 is a table showing another example of the allocation of the candidate substance and the added amount of the candidate substance to each well.

DESCRIPTION OF THE PREFERRED EMBODIMENTS

[First Embodiment]

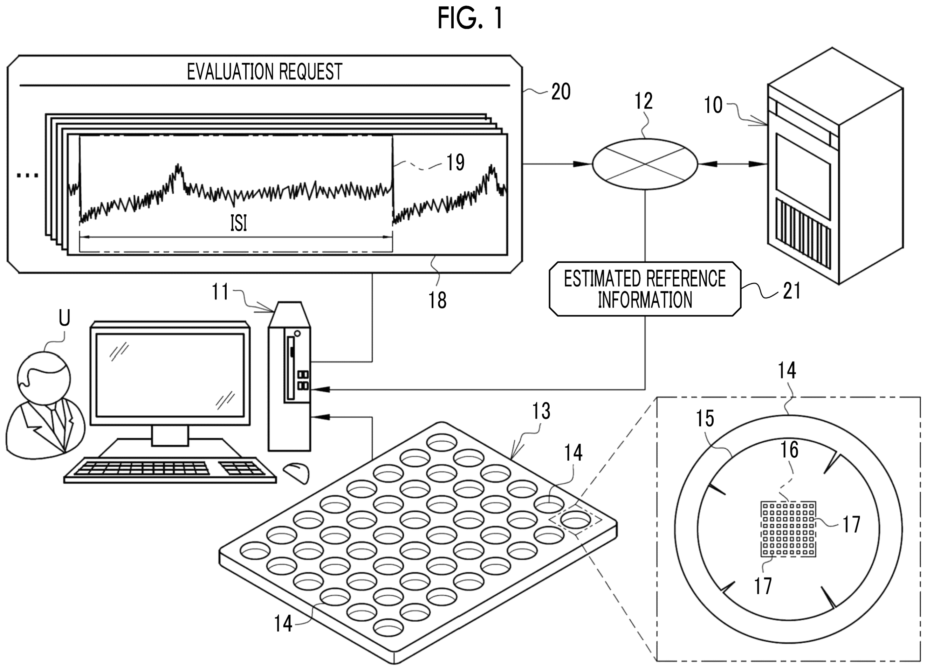

As shown in FIG. 1 as an example, a drug discovery support server 10 is connected to a user terminal 11 via a network 12. The drug discovery support server 10 is an example of a "drug discovery support apparatus" according to the technology of the present disclosure. The user terminal 11 is installed in, for example, a pharmaceutical company that develops drugs or an organization that is contracted by a pharmaceutical company to conduct drug development operations, that is, a contract research organization (CRO). The user terminal 11 is operated by a user U who is involved in the development of drugs in the pharmaceutical company or the contract research organization. The network 12 is, for example, a wide area network (WAN) such as the Internet or a public communication network. In addition, in FIG. 1, only one user terminal 11 is connected to the drug discovery support server 10. However, in practice, a plurality of user terminals 11 of a plurality of pharmaceutical companies or a plurality of contract research organizations are connected to the drug discovery support server 10.

A well plate 13 is connected to the user terminal 11. The well plate 13 has a plurality of wells 14. The wells 14 are arranged at equal intervals along the vertical and horizontal directions. FIG. 1 shows the well plate 13 having 48 (= 8 × 6) wells 14. Further, the number of wells 14 is not limited to 48 given as an example and may be 24, 96, or the like.

The well 14 is a cylindrical recess with an open top. The well 14 is filled with a culture medium, and iPS myocardial cells 15, which are myocardial cells derived from human iPS cells, are cultured in the well 14. The iPS myocardial cell 15 has a sheet shape and grows until the iPS myocardial cell 15 can beat spontaneously. Further, in order to culture the iPS myocardial cell 15 under a certain environment, the well plate 13 is placed in a constant-temperature tank (not shown).

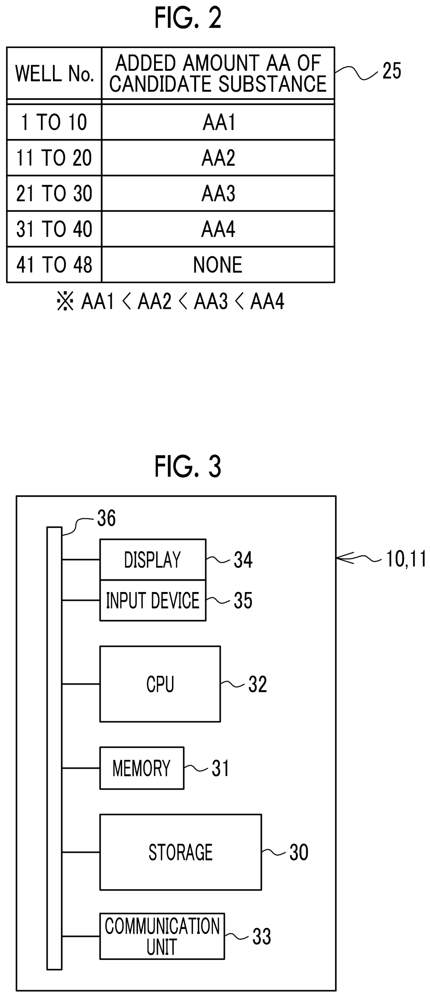

As shown in a table 25 of FIG. 2 as an example, a candidate substance for a drug is added to the wells 14 numbered 1 to 40 among the 48 wells 14. More specifically, an amount AA1 of candidate substance is added to the wells 14 numbered 1 to 10, and an amount AA2 of candidate substance is added to the wells 14 numbered 11 to 20. In addition, an amount AA3 of candidate substance is added to the wells 14 numbered 21 to 30, and an amount AA4 of candidate substance is added to the wells 14 numbered 31 to 40. A magnitude relationship between the added amounts AA1 to AA4 is AA1 < AA2 < AA3 < AA4. The candidate substance is not added to the wells 14 numbered 41 to 48. As described above, the candidate substance is added to the iPS myocardial cell 15 while the added amount AA is changed. In addition, the added amount AA is not limited to the four types given as an example. For example, two types of added amounts AA or ten types of added amounts AA may be used.

Returning to FIG. 1, a planar microelectrode array 16 is provided on a bottom surface of the well 14. The planar microelectrode array 16 is composed of a plurality of microelectrodes 17 that are arranged in a square shape. FIG. 1 shows the planar microelectrode array 16 having 64 (= 8 × 8) microelectrodes 17. The microelectrodes 17 are numbered like the wells 14. The microelectrode 17 is an example of an "electrode" according to the technology of the present disclosure. In addition, the number of microelectrodes 17 is not limited to 64 given as an example and may be 16, 100, or the like.

The microelectrodes 17 are exposed at the bottom surface of the well 14 and come into contact with the iPS myocardial cells 15. The microelectrodes 17 are connected to a potential measurement circuit (not shown). The potential measurement circuit measures an electric signal from each of the plurality of microelectrodes 17, that is, the extracellular potential of the iPS myocardial cell 15. Therefore, the extracellular potential is measured for each of the plurality of microelectrodes 17, in this example, 64 microelectrodes 17. A method of measuring the extracellular potential using the planar microelectrode array 16 is very simple and inexpensive as compared to a method called a manual patch clamp in which electrodes are directly inserted into the iPS myocardial cell 15 to measure the extracellular potential.

The measurement result of the extracellular potential by the potential measurement circuit is input to the user terminal 11. In the user terminal 11, a first fluctuation waveform 18, which is a fluctuation waveform indicating a temporal change in the measurement result of the extracellular potential, is generated. Here, the measurement of the extracellular potential is performed for a preset period, for example, 10 minutes. Therefore, the first fluctuation waveform 18 represents a temporal change in the measurement result of the extracellular potential for the preset period. The number of the well 14 and the number of the microelectrode 17 where the extracellular potential has been measured are stored in the first fluctuation waveform 18 in association with each other.

Several pulses resulting from the beating of the iPS myocardial cell 15 appear in the first fluctuation waveform 18. Therefore, the first fluctuation waveform 18 has periodicity corresponding to the beating of the iPS myocardial cell 15. In other words, the first fluctuation waveform 18 is a set of a plurality of periodic portions 19 separated by one interspike interval (ISI).

The user terminal 11 transmits an evaluation request 20 to the drug discovery support server 10. The evaluation request 20 is a request for the drug discovery support server 10 to evaluate whether or not the candidate substance for a drug has cardiotoxicity. The evaluation request 20 includes a plurality of first fluctuation waveforms 18. The number of first fluctuation waveforms 18 included in the evaluation request 20 depends on the number of wells 14 and the number of microelectrodes 17. In the present example, since the number of wells 14 is 48 and the number of microelectrodes 17 is 64, the evaluation request 20 includes 3072 (= 48 × 64) first fluctuation waveforms 18. Further, the evaluation request 20 also includes, for example, terminal identification data (ID) for uniquely identifying the user terminal 11 which is a transmission source of the evaluation request 20, which is not shown.

In a case where the evaluation request 20 is received, the drug discovery support server 10 derives estimated reference information 21. The drug discovery support server 10 distributes the estimated reference information 21 to the user terminal 11 which is the transmission source of the evaluation request 20. In a case where the estimated reference information 21 is received, the user terminal 11 provides the estimated reference information 21 for viewing by the user U.

The estimated reference information 21 is information referred to by the user U to estimate the mechanism of action of the candidate substance. The mechanism of action of the candidate substance refers to, for example, in which of a plurality of ion channels present in the iPS myocardial cell 15 the flow of ions is inhibited or activated by the candidate substance, resulting in QT prolongation, in a case where the QT prolongation occurs in the iPS myocardial cell 15.

The plurality of ion channels present in the iPS myocardial cell 15 are three channels of an hERG channel through which K ions flow, an Na channel through which Na ions flow, and a Ca channel through which Ca ions flow. These ion channels play a very important role in the beating of the iPS myocardial cell 15. Therefore, it is considered that QT prolongation, which is a cause of the fatal arrhythmia, occurs due to the inhibition or activation of the flow of each ion in these ion channels.

As shown in FIG. 3 as an example, computers constituting the drug discovery support server 10 and the user terminal 11 basically have the same configuration and comprise a storage 30, a memory 31, a central processing unit (CPU) 32, a communication unit 33, a display 34, and an input device 35. These components are connected to each other via a bus line 36.

The storage 30 is a hard disk drive that is built in the computers constituting the drug discovery support server 10 and the user terminal 11 or that is connected to the computers via a cable or a network. Alternatively, the storage 30 is a disk array in which a plurality of hard disk drives are connected in series. The storage 30 stores a control program, such as an operating system, various application programs (hereinafter, referred to as application programs (APs)), various types of data associated with these programs, and the like. In addition, a solid state drive may be used instead of the hard disk drive.

The memory 31 is a work memory for the CPU 32 to execute processes. The CPU 32 loads the program stored in the storage 30 into the memory 31 and executes a process corresponding to the program. In this way, the CPU 32 controls the overall operation of each unit of the computer. In addition, the CPU 32 is an example of a "processor" according to the technology of the present disclosure. Further, the memory 31 may be provided in the CPU 32.

The communication unit 33 is a network interface that controls the transmission of various types of information via the network 12 or the like. The display 34 displays various screens. The various screens have operation functions by a graphical user interface (GUI). The computers constituting the drug discovery support server 10 and the user terminal 11 receive the input of an operation instruction from the input device 35 through the various screens. The input device 35 is a keyboard, a mouse, a touch panel, a microphone for voice input, or the like.

Further, in the following description, in order to distinguish the units of the computers, a subscript "A" is added to the reference numerals of the respective units (the storage 30 and the CPU 32) of the computer constituting the drug discovery support server 10, and a subscript "B" is added to the reference numerals of the respective units (the storage 30, the CPU 32, the display 34, and the input device 35) of the computer constituting the user terminal 11.

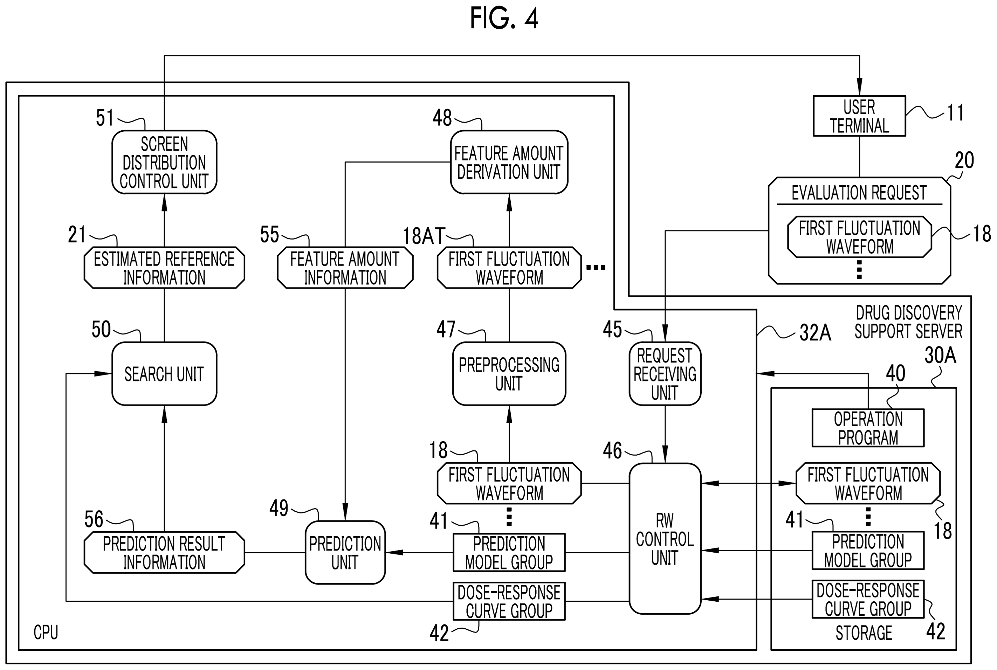

As shown in FIG. 4 as an example, an operation program 40 is stored in the storage 30A of the drug discovery support server 10. The operation program 40 is an AP for causing the computer to function as the drug discovery support server 10. That is, the operation program 40 is an example of a "program for operating a drug discovery support apparatus" according to the technology of the present disclosure. The storage 30A also stores a prediction model group 41, a dose-response curve group 42, and the like.

In a case where the operation program 40 is started, the CPU 32A of the computer constituting the drug discovery support server 10 functions as a request receiving unit 45, a read/write (hereinafter, abbreviated as RW) control unit 46, a preprocessing unit 47, a feature amount derivation unit 48, a prediction unit 49, a search unit 50, and a screen distribution control unit 51 in cooperation with the memory 31 and the like.

The request receiving unit 45 receives various requests including the evaluation request 20 from the user terminal 11. In a case where the evaluation request 20 is received, the request receiving unit 45 outputs the first fluctuation waveform 18 included in the evaluation request 20 to the RW control unit 46. In addition, the request receiving unit 45 outputs the terminal ID of the user terminal 11 included in the evaluation request 20 to the screen distribution control unit 51, which is not shown.

The RW control unit 46 controls the storage of various types of data in the storage 30A and the readout of various types of data from the storage 30A. In particular, the RW control unit 46 controls the storage of the first fluctuation waveform 18 in the storage 30A and the readout of the first fluctuation waveform 18 from the storage 30A. The RW control unit 46 outputs the read-out first fluctuation waveform 18 to the preprocessing unit 47. In addition, the RW control unit 46 reads out the prediction model group 41 from the storage 30A and outputs the read-out prediction model group 41 to the prediction unit 49. Further, the RW control unit 46 reads out the dose-response curve group 42 from the storage 30A and outputs the read-out dose-response curve group 42 to the search unit 50.

The preprocessing unit 47 performs preprocessing on the first fluctuation waveform 18. The preprocessing unit 47 outputs the first fluctuation waveform 18 subjected to the preprocessing (hereinafter, referred to as a first fluctuation waveform 18AT) to the feature amount derivation unit 48.

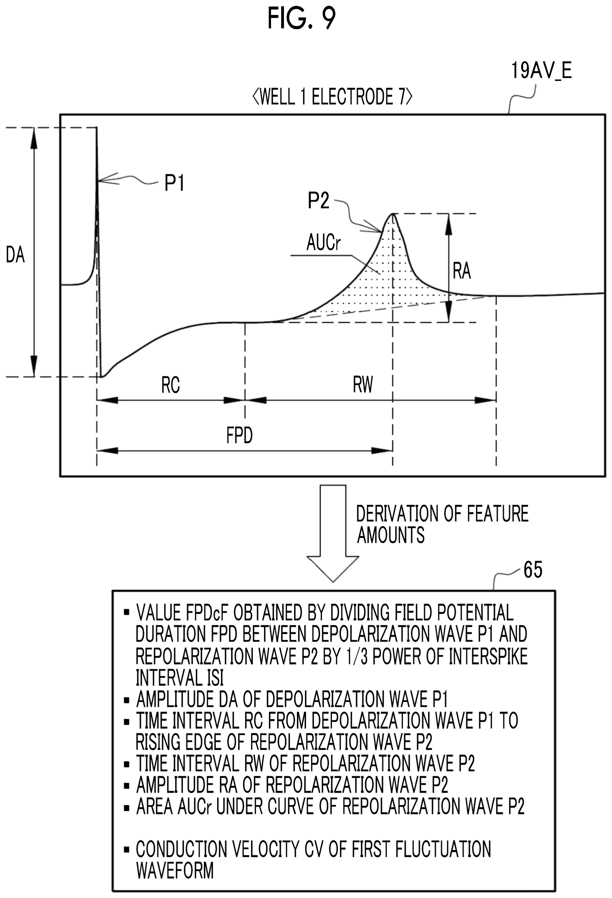

The feature amount derivation unit 48 derives a plurality of types of feature amounts 65 from the first fluctuation waveform 18AT using, for example, a machine learning model that outputs the feature amounts 65 (see FIG. 9) in a case where the first fluctuation waveform 18AT is input. The feature amount derivation unit 48 generates feature amount information 55 obtained by summarizing the plurality of types of derived feature amounts 65 and outputs the feature amount information 55 to the prediction unit 49.

In the prediction unit 49, the prediction model group 41 predicts the degree of action of the candidate substance on each ion channel of the iPS myocardial cell 15 based on the feature amounts 65 of the feature amount information 55. The prediction unit 49 generates prediction result information 56 obtained by summarizing the prediction results of the degree of action of the candidate substance on each ion channel of the iPS myocardial cell 15 and outputs the prediction result information 56 to the search unit 50.

The search unit 50 searches for a dose-response curve suitable for the prediction result information 56 based on the dose-response curve group 42. The search unit 50 derives an index value indicating a dose-response relationship of the candidate substance from the searched dose-response curve. The search unit 50 generates the estimated reference information 21 corresponding to the index value and outputs the estimated reference information 21 to the screen distribution control unit 51.

The screen distribution control unit 51 performs control to distribute various screens to the user terminal 11. Specifically, the screen distribution control unit 51 distributes and outputs the various screens to the user terminal 11, which is the transmission source of various requests, in the form of screen data for web distribution created using a markup language such as Extensible Markup Language (XML). In this case, the screen distribution control unit 51 specifies the user terminal 11, which is the transmission source of the various requests, based on the terminal ID from the request receiving unit 45. Further, instead of XML, another data description language, such as JavaScript (registered trademark) Object Notation (JSON), may be used.

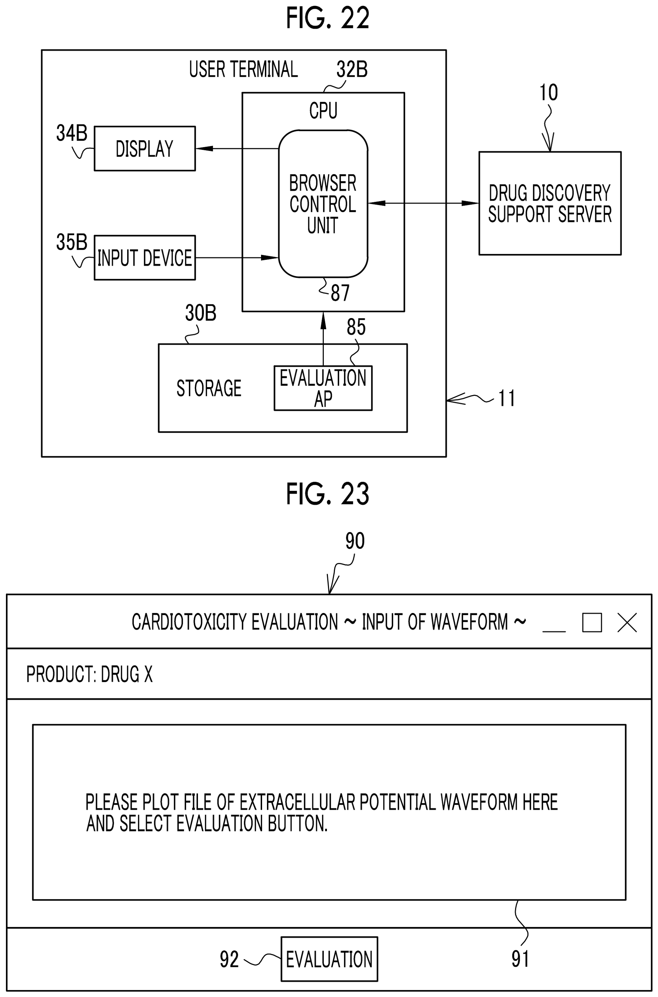

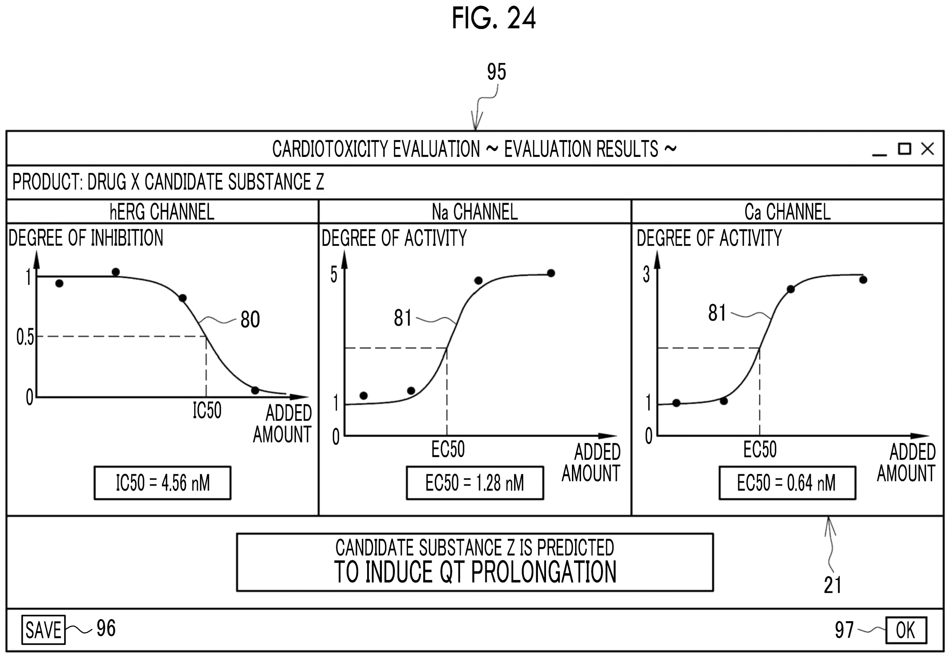

The various screens include a waveform input screen 90 (see FIG. 23) for inputting the first fluctuation waveform 18, an evaluation result display screen 95 (see FIG. 24) for presenting the estimated reference information 21 to the user U, and the like. Further, in addition to these processing units 45 to 51, an instruction receiving unit that receives various operation instructions from the input device 35 is constructed in the CPU 32A.

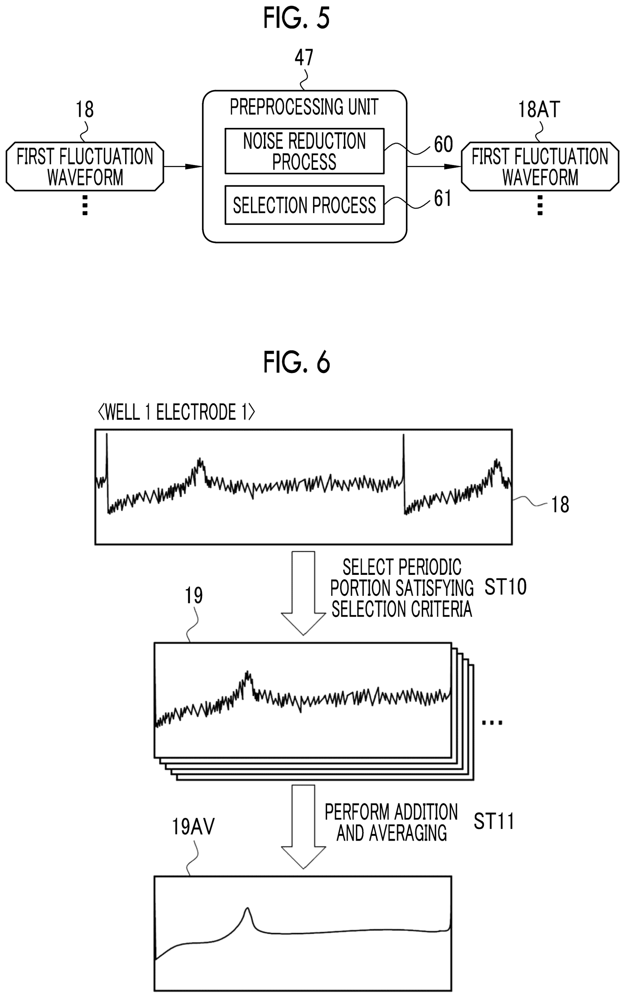

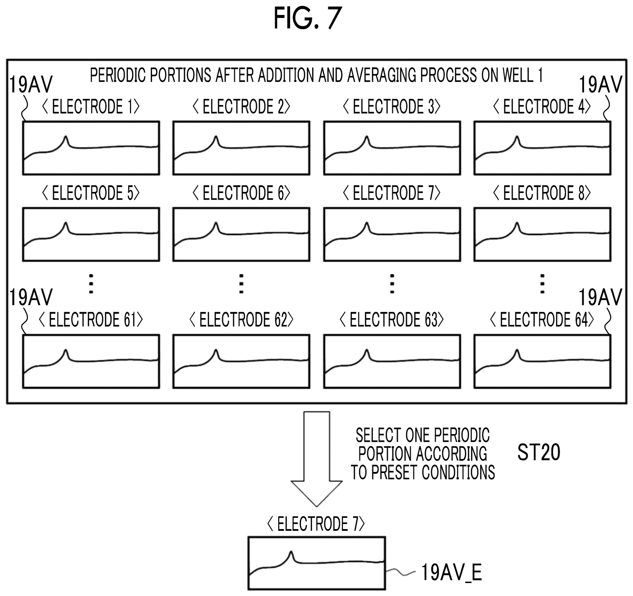

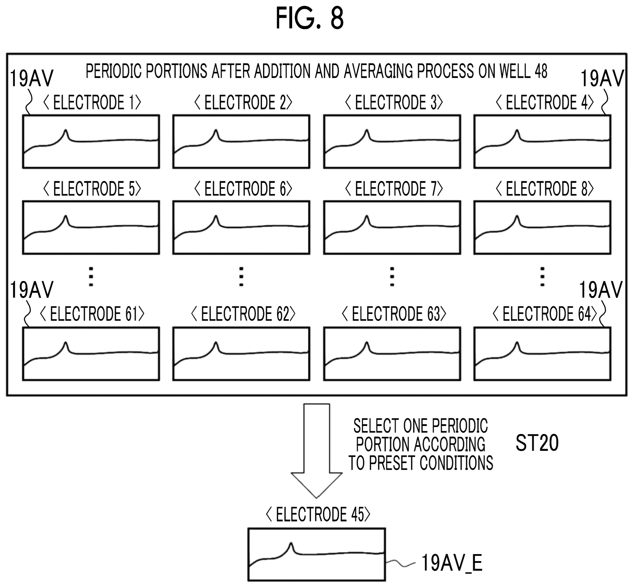

As shown in FIG. 5 as an example, the preprocessing unit 47 performs a noise reduction process 60 and a selection process 61 as the preprocessing on the first fluctuation waveform 18. The noise reduction process 60 is a process shown in FIG. 6 as an example, and the selection process 61 is a process shown in FIGS. 7 and 8 as an example.

As shown in FIG. 6, the preprocessing unit 47 performs the noise reduction process 60 according to the following procedure. First, the preprocessing unit 47 extracts a plurality of periodic portions 19 from the first fluctuation waveform 18 measured in a certain microelectrode 17 of a certain well 14. Then, a set number of periodic portions 19 satisfying preset selection criteria are selected from the plurality of periodic portions 19 (Step ST10). The selection criteria are, for example, that the beat interval ISI is within a set range, the maximum amplitude is equal to or less than a set value, and/or the S/N ratio is equal to or less than a set value. The set number is, for example, 30.

The preprocessing unit 47 adds and averages the selected set number of periodic portions 19 to obtain a periodic portion 19AV (Step ST11). Then, the noise reduction process 60 is ended. That is, the noise reduction process 60 is a process of adding and averaging the plurality of periodic portions 19 of the first fluctuation waveform 18. The preprocessing unit 47 repeats the processes in Steps ST10 and ST11 until there are no more first fluctuation waveforms 18 that have not been subjected to the noise reduction process 60. In other words, the preprocessing unit 47 performs the noise reduction process 60 on all of the first fluctuation waveforms 18. Therefore, the periodic portions 19AV corresponding to the number of first fluctuation waveforms 18 are generated. FIG. 6 shows an example in which the noise reduction process 60 is performed on the first fluctuation waveform 18 measured in the microelectrode 17 numbered 1 in the well 14 numbered 1. Further, in addition to or instead of the addition and averaging process, for example, a smoothing process using a low-pass filter may be performed as the noise reduction process 60 on the first fluctuation waveform 18.

As shown in FIGS. 7 and 8, the preprocessing unit 47 selects one periodic portion 19AV_E from the plurality of periodic portions 19AV generated from the first fluctuation waveforms 18 measured in each microelectrode 17 of a certain well 14. In this case, the preprocessing unit 47 selects the periodic portion 19AV_E according to preset conditions (Step ST20). The periodic portion 19AV_E is an object from which the feature amount 65 is derived by the feature amount derivation unit 48. That is, the periodic portion 19AV_E is an example of a "first fluctuation waveform from which a feature amount is derived" according to the technology of the present disclosure. As described above, the selection process 61 is a process of selecting a so-called golden channel.

Here, the selection of the periodic portion 19AV_E according to the preset conditions means, for example, selection using a method disclosed in WO2022/176310A. The method disclosed in WO2022/176310A is a method that repeatedly performs clustering analysis between a plurality of waveforms to be selected (a plurality of periodic portions 19AV in the present example) and a plurality of training waveforms annotated with labels indicating whether or not the training waveforms are ideal to select one waveform (in the present example, the periodic portion 19AV_E) from the plurality of waveforms to be selected. In addition, conditions may be set based on a feature amount obtained from an ideal waveform, and the periodic portion 19AV_E satisfying the conditions may be selected.

The preprocessing unit 47 repeats the process in Step ST20 until there are no more wells 14 that have not been subjected to the selection process 61. In other words, the preprocessing unit 47 performs the selection process 61 on all of the wells 14. Therefore, the periodic portions 19AV_E corresponding to the number of wells 14 are selected. The preprocessing unit 47 outputs the periodic portions 19AV_E corresponding to the number of wells 14 as the first fluctuation waveforms 18AT to the feature amount derivation unit 48. FIG. 7 shows an example in which the periodic portion 19AV related to the microelectrode 17 numbered 7 is selected as the periodic portion 19AV_E from a plurality of periodic portions 19AV related to the respective microelectrodes 17 of the well 14 numbered 1. In addition, FIG. 8 shows an example in which the periodic portion 19AV related to the microelectrode 17 numbered 45 is selected as the periodic portion 19AV_E from a plurality of periodic portions 19AV related to the respective microelectrodes 17 of the well 14 numbered 48. According to FIG. 2, the well 14 numbered 48 is the well 14 to which the candidate substance is not added. Therefore, the periodic portion 19AV_E selected in FIG. 8 is an example of a "reference first fluctuation waveform" according to the technology of the present disclosure.

As shown in FIG. 9 as an example, the feature amount derivation unit 48 derives the following as the feature amount 65 from the periodic portion 19AV_E. That is, Field Potential Duration (FPD) cF, Depolarization Amplitude (DA), Repolarization Center (RC), Repolarization Width (RW), Repolarization Amplitude (RA), Area Under Curve of the repolarization wave (AUCr), and Conduction Velocity (CV) are derived.

FPDcF is a value obtained by dividing the field potential duration (FPD) between a depolarization wave P1 and a repolarization wave P2, which are pulses caused by the beating of the iPS myocardial cell 15, by the 1/3 power of the interspike interval ISI. That is, $FPDcF = FPD/(ISI)^{1/3}$. DA is the amplitude of the depolarization wave P1. RC is a time interval (repolarization center) from the depolarization wave P1 to the rising edge of the repolarization wave P2. RW is a time interval (repolarization width) of the repolarization wave P2. RA is the amplitude of the repolarization wave P2. AUCr is the area under the curve of the repolarization wave P2. CV is the conduction velocity of the first fluctuation waveform 18. FPDcF (FPD), DA, RC, RW, RA, and AUCr are feature amounts listed in Non-Patent Document 2. CV is a feature amount described in Non-Patent Document 1. CV is calculated based on the disposition position of the microelectrode 17.

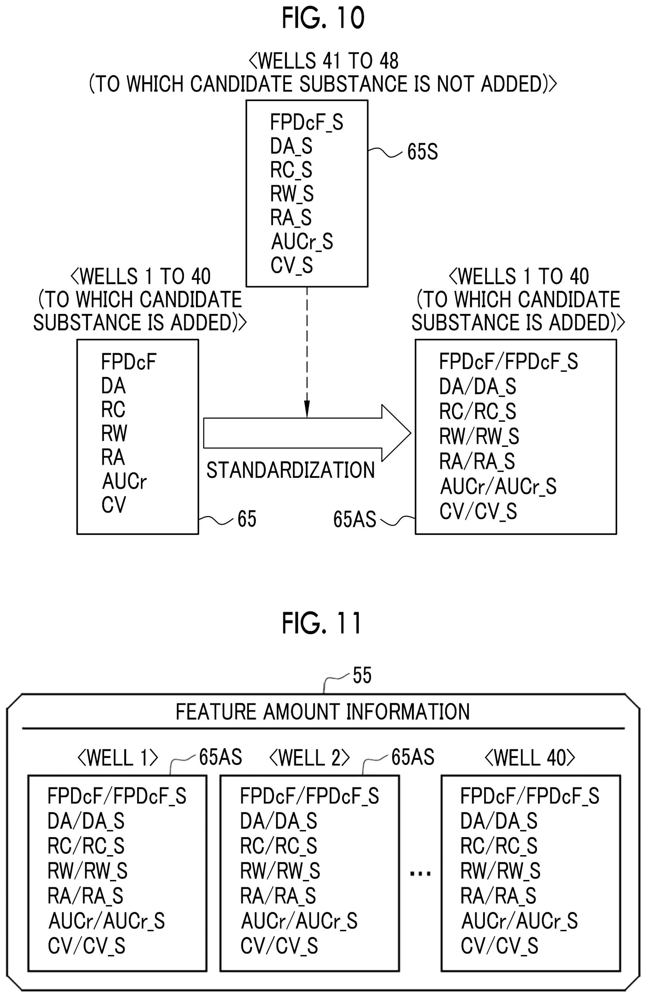

As shown in FIG. 10 as an example, the feature amount derivation unit 48 divides the feature amounts 65 derived from the periodic portions 19AV_E of the wells 14 numbered 1 to 40, to which the candidate substance has been added, by reference feature amounts 65S derived from the periodic portions 19AV_E of the wells 14 numbered 41 to 48, to which the candidate substance has not been added, to standardize the feature amounts 65, thereby obtaining feature amounts 65AS. As shown in FIG. 11 as an example, the feature amount information 55 is a set of the feature amounts 65AS of the wells 14 numbered 1 to 40. In addition, the reference feature amount 65S is, for example, a representative value such as an average value or a median value of the feature amounts 65 derived from the periodic portions 19AV_E of the wells 14 numbered 41 to 48.

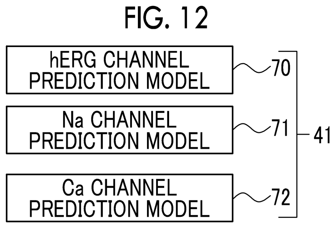

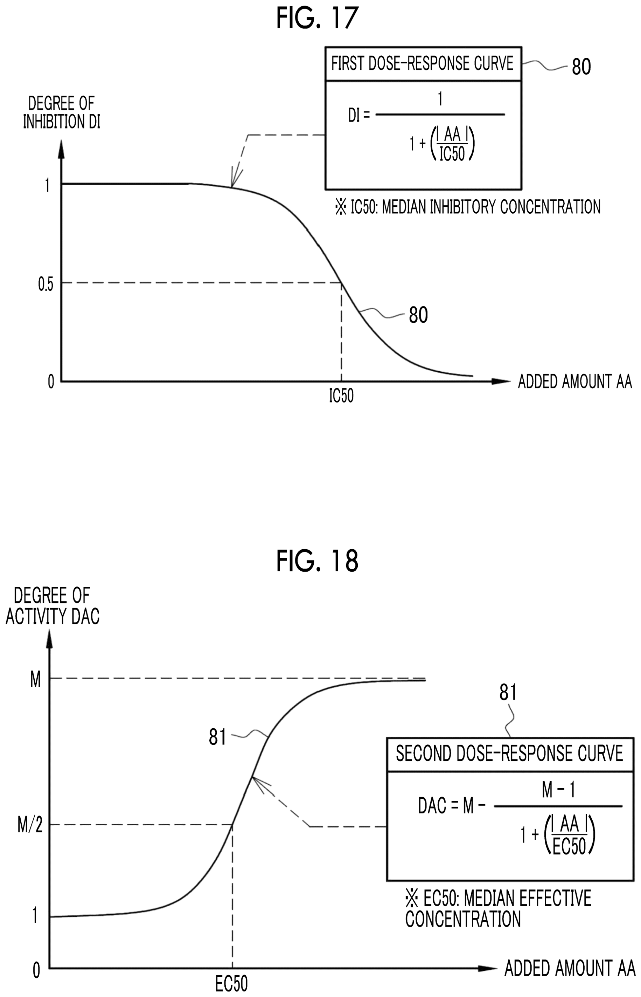

As shown in FIG. 12 as an example, the prediction model group 41 is composed of an hERG channel prediction model 70, an Na channel prediction model 71, and a Ca channel prediction model 72. The hERG channel prediction model 70 predicts the degree of inhibition (hereinafter, may be referred to as a degree of inhibition DI, see FIG. 17) indicating the degree to which the flow of K ions in the hERG channel is inhibited by the candidate substance or the degree of activity (hereinafter, may be referred to as a degree of activity DAC, see FIG. 18) indicating the degree to which the flow of K ions in the hERG channel is activated by the candidate substance. The Na channel prediction model 71 predicts the degree of inhibition indicating the degree to which the flow of Na ions in the Na channel is inhibited by the candidate substance or the degree of activity indicating the degree to which the flow of Na ions in the Na channel is activated by the candidate substance. The Ca channel prediction model 72 predicts the degree of inhibition indicating the degree to which the flow of Ca ions in the Ca channel is inhibited by the candidate substance or the degree of activity indicating the degree to which the flow of Ca ions in the Ca channel is activated by the candidate substance. Each of the prediction models 70 to 72 is, for example, a machine learning model constructed by a machine learning method such as a neural network. In addition, each of the prediction models 70 to 72 is a machine learning model corresponding to multi-label regression using a method such as a regressor chain. Therefore, each of the prediction models 70 to 72 is a model that takes into account the correlation between the ion channels.

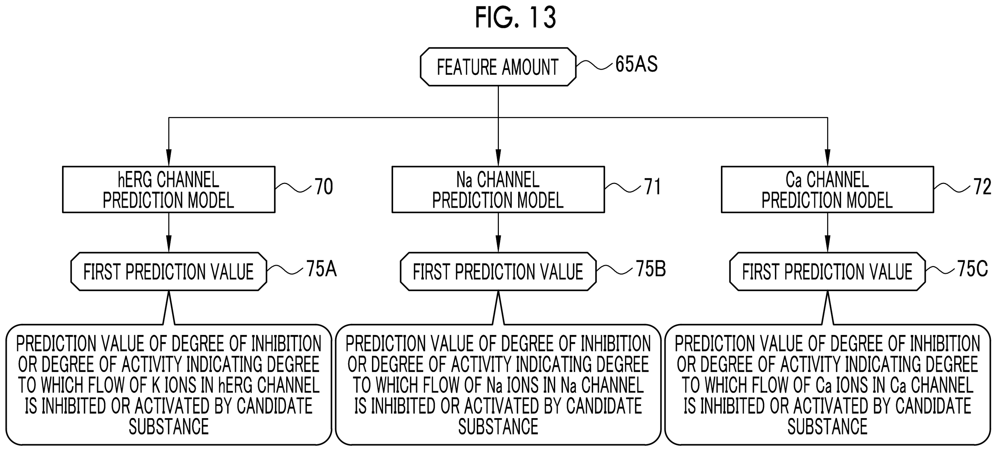

As shown in FIG. 13 as an example, in the prediction unit 49, the feature amount 65AS is input to each of the prediction models 70 to 72, and first prediction values 75A, 75B, and 75C are output from the prediction models 70 to 72, respectively. The first prediction value 75A output from the hERG channel prediction model 70 is a prediction value of the degree of inhibition indicating the degree to which the flow of K ions in the hERG channel is inhibited by the candidate substance or the degree of activity indicating the degree to which the flow of K ions in the hERG channel is activated by the candidate substance. The first prediction value 75B output from the Na channel prediction model 71 is a prediction value of the degree of inhibition indicating the degree to which the flow of Na ions in the Na channel is inhibited by the candidate substance or the degree of activity indicating the degree to which the flow of Na ions in the Na channel is activated by the candidate substance. The first prediction value 75C output from the Ca channel prediction model 72 is a prediction value of the degree of inhibition indicating the degree to which the flow of Ca ions in the Ca channel is inhibited by the candidate substance or the degree of activity indicating the degree to which the flow of Ca ions in the Ca channel is activated by the candidate substance. The first prediction values 75A to 75C are values that are greater than 0 and less than 1 in a case where the flow of ions is predicted to be inhibited by the candidate substance and are values that are greater than 1 in a case where the flow of ions is predicted to be activated by the candidate substance. In a case where the flow of ions is predicted to be neither inhibited nor activated by the candidate substance, the first prediction values 75A to 75C are 1. Further, in the following description, in a case where it is not necessary to particularly distinguish the first prediction values 75A to 75C, the first prediction values 75A to 75C are simply referred to as first prediction values 75.

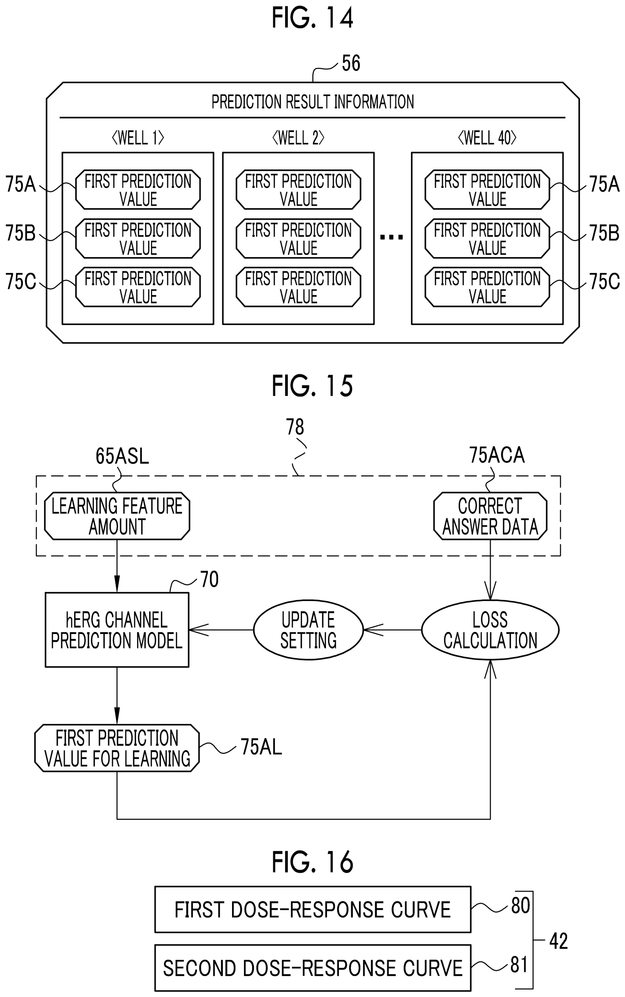

As shown in FIG. 14 as an example, the prediction result information 56 is a set of the first prediction values 75A to 75C for each of the wells 14 numbered 1 to 40. That is, the prediction result information 56 is a set of the first prediction values 75A to 75C for each ion channel and for each added amount.

Further, the prediction unit 49 also predicts whether or not QT prolongation will occur in the iPS myocardial cell 15 due to the addition of the candidate substance, using a prediction model different from each of the prediction models 70 to 72. The prediction result information 56 also includes the result of predicting whether or not QT prolongation will occur in the iPS myocardial cell 15. The feature amount 65AS is input to the prediction model that predicts whether or not QT prolongation will occur in the iPS myocardial cell 15, similarly to each of the prediction models 70 to 72. Alternatively, the first prediction values 75A to 75C are input.

As shown in FIG. 15 as an example, the hERG channel prediction model 70 is trained with learning data 78. The learning data 78 is a set of a learning feature amount 65ASL and correct answer data 75ACA. The learning feature amount 65ASL is the feature amount 65AS of the candidate substance that was actually tested in the past. The correct answer data 75ACA is a result of actually measuring the degree of inhibition or the degree of activity of the candidate substance, which is a provision source of the learning feature amount 65ASL, in the test actually performed in the past.

The learning feature amount 65ASL is input to the hERG channel prediction model 70. Then, the hERG channel prediction model 70 outputs a first prediction value 75AL for learning. The first prediction value 75AL for learning is compared with the correct answer data 75ACA, and loss calculation of the hERG channel prediction model 70 using a loss function is performed based on the comparison result. Then, the update setting of internal parameters, such as filter coefficients, of the hERG channel prediction model 70 is performed according to the result of the loss calculation, and the hERG channel prediction model 70 is updated according to the update setting.

The series of processes including the input of the learning feature amount 65ASL to the hERG channel prediction model 70, the output of the first prediction value 75AL for learning from the hERG channel prediction model 70, the loss calculation, the update setting, and the update of the hERG channel prediction model 70 is repeatedly performed while the learning data 78 is changed. Then, in a case where the prediction accuracy of the first prediction value 75AL for learning with respect to the correct answer data 75ACA reaches a preset level, the repetition of the series of processes is ended. Then, the hERG channel prediction model 70 in which the prediction accuracy has reached the preset level is stored in the storage 30A. In addition, regardless of the prediction accuracy, the training may be ended in a case in which the series of processes has been repeated a predetermined number of times. Further, the training of the hERG channel prediction model 70 may be continued even after the hERG channel prediction model 70 is stored in the storage 30A.

The processes in the training phase of the hERG channel prediction model 70 have been described above. However, the processes in the training phase of the Na channel prediction model 71 and the Ca channel prediction model 72 are basically the same as the processes in the training phase of the hERG channel prediction model 70, except that the learning data 78 is changed. Therefore, the showing and description of the Na channel prediction model 71 and the Ca channel prediction model 72 are omitted.

As shown in FIG. 16 as an example, the dose-response curve group 42 is composed of a first dose-response curve 80 and a second dose-response curve 81. The first dose-response curve 80 is a dose-response curve in a case where the flow of ions in the ion channel is inhibited by the candidate substance. The second dose-response curve 81 is a dose-response curve in a case where the flow of ions in the ion channel is activated by the candidate substance.

As shown in FIG. 17 as an example, the first dose-response curve 80 is a logistic curve represented by the following Expression (1).

$$DI = \frac{1}{1 + \left( \frac{|AA|}{IC50} \right)} \qquad \cdots (1)$$

Here, DI is the degree of inhibition, AA is the added amount of candidate substance, and IC50 is the median inhibitory concentration. In a two-dimensional space in which the degree of inhibition DI is the vertical axis and the added amount AA is the horizontal axis, the first dose-response curve 80 is a reverse S-shaped curve in which the degree of inhibition DI gradually decreases from 1 to 0 with an increase in the added amount AA.

In addition, as shown in FIG. 18 as an example, the second dose-response curve 81 is a logistic curve represented by the following Expression (2).

$$DAC = M - \frac{M-1}{1 + \left( \frac{|AA|}{EC50} \right)} \qquad \cdots (2)$$

Here, DAC is the degree of activity, AA is the added amount of candidate substance as in the case of the first dose-response curve 80, EC50 (median effective concentration) is the median effective concentration, and M is the value of the asymptote. In a two-dimensional space in which the degree of activity DAC is the vertical axis and the added amount AA is the horizontal axis, the second dose-response curve 81 is an S-shaped curve in which the degree of activity DAC gradually increases from 1 to M with an increase in the added amount AA.

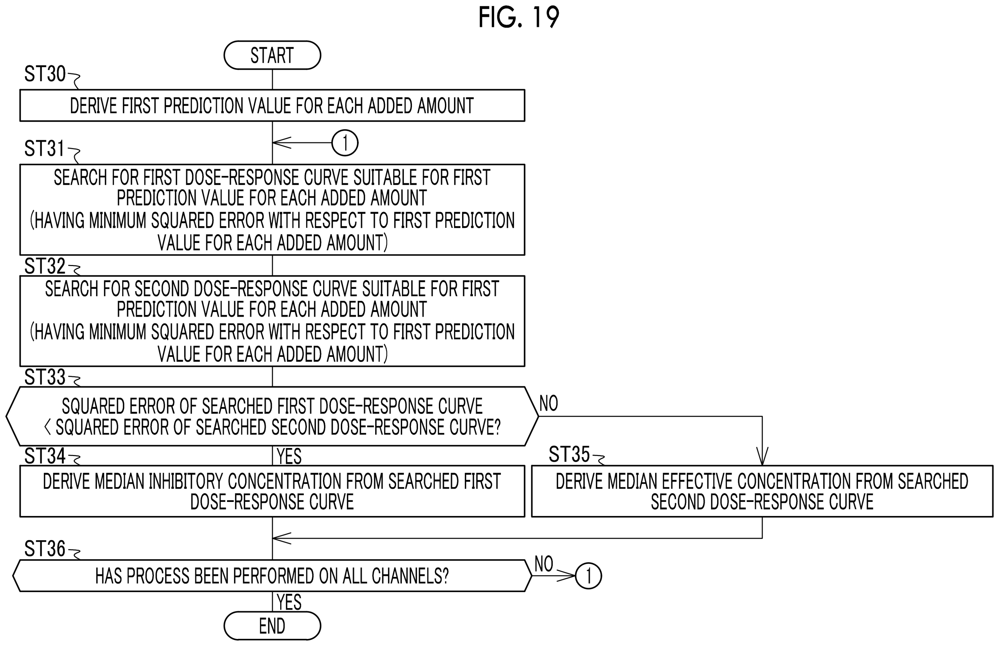

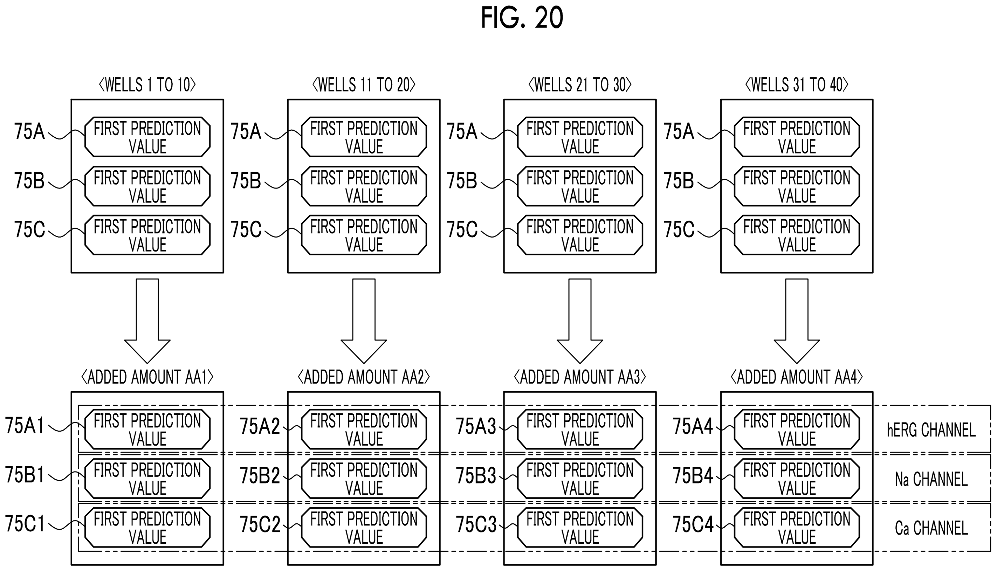

The search unit 50 performs a process according to the procedure shown in FIG. 19 as an example. First, the search unit 50 derives the first prediction values 75A to 75C for each added amount AA (Step ST30). More specifically, as shown in FIG. 20 as an example, the search unit 50 sets the representative values of the first prediction values 75A to 75C related to the wells 14 numbered 1 to 10 as first prediction values 75A1 to 75C1 of the added amount AA1, respectively, and sets the representative values of the first prediction values 75A to 75C related to the wells 14 numbered 11 to 20 as first prediction values 75A2 to 75C2 of the added amount AA2, respectively. Similarly, the search unit 50 sets the representative values of the first prediction values 75A to 75C related to the wells 14 numbered 21 to 30 as the first prediction values 75A3 to 75C3 of the added amount AA3, respectively, and sets the representative values of the first prediction values 75A to 75C related to the wells 14 numbered 31 to 40 as first prediction values 75A4 to 75C4 of the added amount AA4, respectively. As a result, the first prediction values 75 for each ion channel and for each added amount are obtained. In addition, the representative value is, for example, an average value, a median value, or the like.

Returning to FIG. 19, the search unit 50 searches for the first dose-response curve 80 suitable for the first prediction values 75A to 75C for each added amount of a certain ion channel (Step ST31). Specifically, the search unit 50 calculates the squared error between the first dose-response curve 80 and the first prediction values 75A to 75C for each added amount while changing the IC50 of the first dose-response curve 80 each time the IC50 of the first dose-response curve 80 is changed. Then, a first dose-response curve 80 having the IC50 at which the squared error is minimized is defined as the first dose-response curve 80 suitable for the first prediction values 75A to 75C for each added amount.

In addition, the search unit 50 searches for the second dose-response curve 81 suitable for the first prediction values 75A to 75C for each added amount of a certain ion channel (Step ST32). Specifically, the search unit 50 calculates the squared error between the second dose-response curve 81 and the first prediction values 75A to 75C for each added amount while changing M and EC50 of the second dose-response curve 81 each time M and EC50 of the second dose-response curve 81 are changed. Then, a second dose-response curve 81 having M and EC50 at which the squared error is minimized is defined as the second dose-response curve 81 suitable for the first prediction values 75A to 75C for each added amount.

Then, the search unit 50 compares the magnitude of the squared error of the first dose-response curve 80 searched for in Step ST31 with the magnitude of the squared error of the second dose-response curve 81 searched for in Step ST32 (Step ST33). In a case where the squared error of the searched first dose-response curve 80 is smaller than the squared error of the searched second dose-response curve 81 (YES in Step ST33), that is, in a case where the first dose-response curve 80 is more suitable for the first prediction values 75A to 75C for each added amount than the second dose-response curve 81, the search unit 50 derives the median inhibitory concentration IC50 from the searched first dose-response curve 80 (Step ST34). The median inhibitory concentration IC50 derived from the first dose-response curve 80 is an example of an "index value" according to the technology of the present disclosure.

On the other hand, in a case where the squared error of the searched second dose-response curve 81 is smaller than the squared error of the searched first dose-response curve 80 (NO in Step ST33), that is, in a case where the second dose-response curve 81 is more suitable for the first prediction values 75A to 75C for each added amount than the first dose-response curve 80, the search unit 50 derives the median effective concentration EC50 from the searched second dose-response curve 81 (Step ST35). The median effective concentration EC50 derived from the second dose-response curve 81 is also an example of the "index value" according to the technology of the present disclosure. The search unit 50 repeats the processes in Steps ST31 to ST35 as long as the median inhibitory concentration IC50 or the median effective concentration EC50 for all of the ion channels has not been derived (NO in Step ST36). In other words, the search unit 50 derives the median inhibitory concentration IC50 or the median effective concentration EC50 for all of the ion channels. The search unit 50 outputs the first prediction value 75, the searched first dose-response curve 80 or second dose-response curve 81, and the derived median inhibitory concentration IC50 or median effective concentration EC50 as the estimated reference information 21 to the screen distribution control unit 51. Further, the search unit 50 also outputs the result of predicting whether or not QT prolongation will occur in the iPS myocardial cell 15 due to the addition of the candidate substance as the estimated reference information 21 to the screen distribution control unit 51. In addition, the estimated reference information 21 may be only the median inhibitory concentration IC50 or only the median effective concentration EC50.

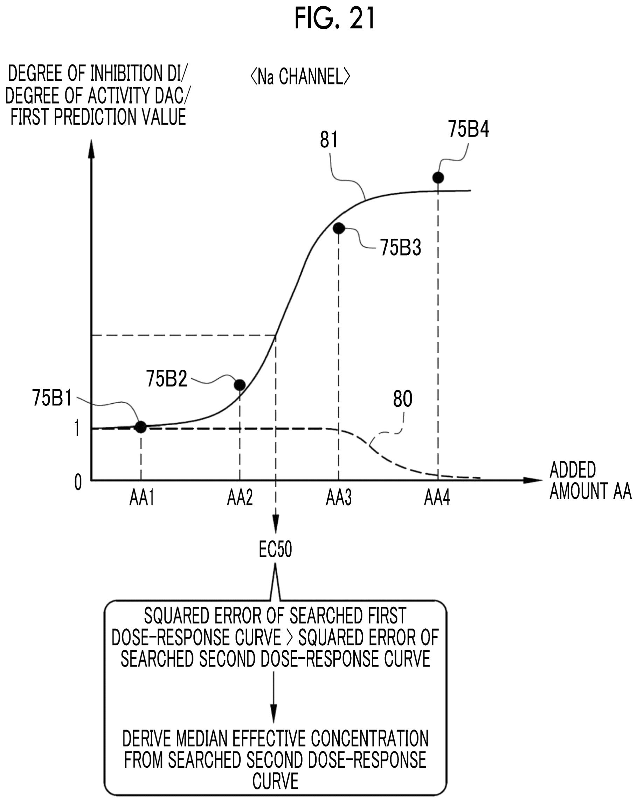

FIG. 21 shows an example in which the Na channel is a target. FIG. 21 shows a case where the squared error of the searched second dose-response curve 81 indicated by a solid line is smaller than the squared error of the searched first dose-response curve 80 indicated by a broken line. In addition, FIG. 21 shows a case where the median effective concentration EC50 is derived from the searched second dose-response curve 81.

As shown in FIG. 22 as an example, an evaluation AP 85 is stored in the storage 30B of the user terminal 11. The evaluation AP 85 is installed in the user terminal 11 by the user U. The evaluation AP 85 is an AP for the drug discovery support server 10 to evaluate whether or not the candidate substance has cardiotoxicity. In a case in which the evaluation AP 85 is started, the CPU 32B of the user terminal 11 functions as a browser control unit 87 in cooperation with the memory 31 and the like. The browser control unit 87 controls the operation of a dedicated web browser of the evaluation AP 85.

The browser control unit 87 reproduces various screens based on various types of screen data from the drug discovery support server 10 and displays the reproduced various screens on the display 34B. In addition, the browser control unit 87 receives various operation instructions input from the input device 35B by the user U through various screens. The browser control unit 87 transmits various requests including the evaluation request 20 to the drug discovery support server 10 in response to the operation instruction.

In a case where the evaluation AP 85 is started, the waveform input screen 90 shown in FIG. 23 as an example is displayed on the display 34B under the control of the browser control unit 87. An input box 91 for the first fluctuation waveform 18 is provided on the waveform input screen 90. A file of the first fluctuation waveform 18 can be dropped in the input box 91. The user U drops a file of a desired first fluctuation waveform 18 in the input box 91 and then selects an evaluation button 92. In a case where the evaluation button 92 is selected, the browser control unit 87 generates the evaluation request 20 including the first fluctuation waveform 18 input to the input box 91 and transmits the generated evaluation request 20 to the drug discovery support server 10.

In addition, in a case where it is evaluated whether or not the candidate substance has cardiotoxicity in the drug discovery support server 10, the evaluation result display screen 95 shown in FIG. 24 as an example is displayed on the display 34B under the control of the browser control unit 87. The estimated reference information 21 is displayed on the evaluation result display screen 95. That is, a graph of the first dose-response curve 80 or the second dose-response curve 81 with the plot of the first prediction value 75, and the median inhibitory concentration IC50 or the median effective concentration EC50 are displayed for each ion channel on the evaluation result display screen 95. In addition, the result of predicting whether or not QT prolongation will occur in the iPS myocardial cell 15 due to the candidate substance is also displayed on the evaluation result display screen 95. As described above, the estimated reference information 21 is presented to the user U in the form of the distribution of the screen data.

A save button 96 and an OK button 97 are provided in a lower part of the evaluation result display screen 95. In a case where the save button 96 is selected, the display content of the evaluation result display screen 95 including the estimated reference information 21 is stored in the storage 30B of the user terminal 11. In a case where the OK button 97 is selected, the display of the evaluation result display screen 95 is turned off.

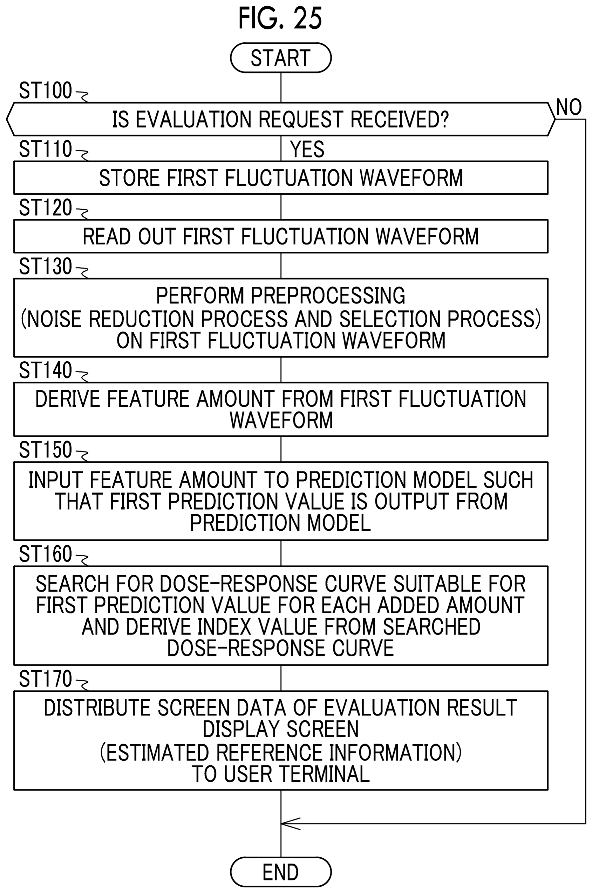

Next, the operation of the above-mentioned configuration will be described with reference to a flowchart shown in FIG. 25 as an example. In a case where the operation program 40 is started in the drug discovery support server 10, the CPU 32A of the drug discovery support server 10 functions as the request receiving unit 45, the RW control unit 46, the preprocessing unit 47, the feature amount derivation unit 48, the prediction unit 49, the search unit 50, and the screen distribution control unit 51 as shown in FIG. 4. In addition, in a case where the evaluation AP 85 is started in the user terminal 11, the CPU 32B of the user terminal 11 functions as the browser control unit 87 as shown in FIG. 22.

The waveform input screen 90 shown in FIG. 23 is displayed on the display 34B of the user terminal 11 under the control of the browser control unit 87. In a case where the user U inputs a file of a desired first fluctuation waveform 18 to the input box 91 and selects the evaluation button 92 on the waveform input screen 90, the evaluation request 20 is transmitted from the browser control unit 87 to the drug discovery support server 10.

In the drug discovery support server 10, the request receiving unit 45 receives the evaluation request 20 (YES in Step ST100). The first fluctuation waveform 18 included in the evaluation request 20 is output from the request receiving unit 45 to the RW control unit 46 and is stored in the storage 30A under the control of the RW control unit 46 (Step ST110). In addition, the terminal ID of the user terminal 11 included in the evaluation request 20 is output from the request receiving unit 45 to the screen distribution control unit 51.

The first fluctuation waveform 18 is read out from the storage 30A by the RW control unit 46 (Step ST120). The first fluctuation waveform 18 is output from the RW control unit 46 to the preprocessing unit 47.

As shown in FIGS. 5 to 8, the preprocessing unit 47 performs the noise reduction process 60 and the selection process 61 as the preprocessing on the first fluctuation waveform 18 (Step ST130). As a result, the first fluctuation waveform 18AT composed of the periodic portions 19AV_E for each well 14 is output from the preprocessing unit 47 to the feature amount derivation unit 48.

As shown in FIGS. 9 and 10, the feature amount derivation unit 48 derives the feature amount 65AS from the periodic portion 19AV_E (Step ST140). Then, the feature amount information 55 shown in FIG. 11, which is a set of the feature amounts 65AS for each well 14, is output from the feature amount derivation unit 48 to the prediction unit 49.

As shown in FIG. 13, in the prediction unit 49, the feature amount 65AS is input to each of the prediction models 70 to 72, and the first prediction values 75A to 75C are output from the prediction models 70 to 72, respectively (Step ST150). Then, the prediction result information 56 shown in FIG. 14, which is a set of the first prediction values 75A to 75C for each well 14, is output from the prediction unit 49 to the search unit 50.

Then, as shown in FIGS. 19 to 21, the search unit 50 searches for the first dose-response curve 80 or the second dose-response curve 81 suitable for the first prediction value 75 for each added amount. Then, the median inhibitory concentration IC50 or the median effective concentration EC50 as the index value is derived from the searched first dose-response curve 80 or second dose-response curve 81 (Step ST160). The estimated reference information 21 including, for example, the searched first dose-response curve 80 or second dose-response curve 81, and the derived median inhibitory concentration IC50 or median effective concentration EC50 is output from the search unit 50 to the screen distribution control unit 51.

The screen distribution control unit 51 generates the screen data of the evaluation result display screen 95 shown in FIG. 24 based on the estimated reference information 21. The screen data of the evaluation result display screen 95 is distributed to the user terminal 11, which is the transmission source of the evaluation request 20, under the control of the screen distribution control unit 51 (Step ST170).

In the user terminal 11, under the control of the browser control unit 87, the screen data of the evaluation result display screen 95 is reproduced, and the reproduced evaluation result display screen 95 is displayed on the display 34B. Therefore, the estimated reference information 21 is presented to the user U.

As described above, the prediction unit 49 of the drug discovery support server 10 acquires the first prediction values 75 for each of a plurality of ion channels present in the iPS myocardial cell 15, which is a myocardial cell derived from the human iPS cell, and for each added amount of the candidate substance for a drug. The first prediction values 75 are output from the prediction models 70 to 72 based on the first fluctuation waveform 18. The first fluctuation waveform 18 is a fluctuation waveform of the extracellular potential of the iPS myocardial cell 15 and is measured in a case where the candidate substance is added to the iPS myocardial cell 15 while the added amount is changed. The first prediction value 75 is a prediction value of the degree of inhibition indicating the degree to which the flow of ions in a plurality of ion channels is inhibited by the candidate substance.

The search unit 50 derives the median inhibitory concentration IC50, which is an index value indicating the dose-response relationship of the candidate substance, for each ion channel, based on the first prediction value 75 for each added amount. The screen distribution control unit 51 distributes the screen data of the evaluation result display screen 95 including the estimated reference information 21 to the user terminal 11 to present the estimated reference information 21 to the user U. The estimated reference information 21 is information corresponding to the median inhibitory concentration IC50, which is an index value, and is referred to in order to estimate the mechanism of action of the candidate substance.

The user U can view the estimated reference information 21 through the evaluation result display screen 95 to understand at a glance the degree to which the flow of ions in each ion channel is inhibited by the candidate substance. Therefore, for example, in a case where QT prolongation occurs in the iPS myocardial cell 15, it is possible to easily estimate the mechanism of action by which the candidate substance induces QT prolongation such as in which of the ion channels the flow of ions is inhibited by the candidate substance, resulting in the QT prolongation. On the contrary, in a case where QT prolongation does not occur in the iPS myocardial cell 15, it is also possible to easily estimate the mechanism of action by which the candidate substance does not induce QT prolongation. In a case where it is possible to easily estimate the mechanism of action of the candidate substance, guidelines for the structure of the candidate substance that does not induce QT prolongation are easily obtained, which makes it possible to significantly promote the development of drugs.

The search unit 50 searches for the first dose-response curve 80 or the second dose-response curve 81 suitable for the first prediction value 75 for each added amount and derives the median inhibitory concentration IC50 or the median effective concentration EC50 from the searched first dose-response curve 80 or second dose-response curve 81. Therefore, it is possible to derive the median inhibitory concentration IC50 or the median effective concentration EC50 with higher reliability.

The first prediction value 75 is a prediction value of the degree of inhibition and is also a prediction value of the degree of activity indicating the degree to which the flow of ions in the ion channel is activated by the candidate substance. In addition, there are two types of dose-response curves: the first dose-response curve 80 in a case where the flow of ions in the ion channel is inhibited by the candidate substance; and the second dose-response curve 81 in a case where the flow of ions in the ion channel is activated by the candidate substance. The search unit 50 derives the median inhibitory concentration IC50 or the median effective concentration EC50 from one of the first dose-response curve 80 and the second dose-response curve 81 which is more suitable for the first prediction value 75 for each added amount. Therefore, it is possible to estimate the mechanism of action of the candidate substance in consideration of the activating action, in addition to the action of inhibiting the flow of ions in the ion channel by the candidate substance. In addition, it is possible to derive the median inhibitory concentration IC50 or the median effective concentration EC50 with higher reliability.

The screen distribution control unit 51 presents the searched first dose-response curve 80 or the searched second dose-response curve 81 as the estimated reference information 21 to the user U. Therefore, the user U can immediately understand in which of the ion channels the flow of ions is inhibited or activated by the candidate substance.

The curve used to search for the dose-response curve is a logistic curve. The logistic curve is generally used to search for a dose-response curve. Therefore, it is possible to easily search for the dose-response curve suitable for the first prediction value 75 for each added amount according to the method of the related art. In addition, the curve used to search for the dose-response curve may be a probit curve or the like.

The first prediction value 75 is obtained by inputting the feature amount 65AS derived from the first fluctuation waveform 18 to the prediction models 70 to 72. Therefore, it is possible to easily improve the prediction accuracy of the first prediction value 75. In addition, instead of or in addition to the feature amount 65AS, the first fluctuation waveform 18 may be input to the prediction models 70 to 72.

The request receiving unit 45 receives the evaluation request 20 to acquire the first fluctuation waveform 18. The feature amount derivation unit 48 derives the feature amount 65AS from the first fluctuation waveform 18. In the prediction unit 49, the feature amount 65AS is input to the prediction models 70 to 72, and the first prediction values 75A to 75C are output from the prediction models 70 to 72, respectively. In this way, the drug discovery support server 10 is solely in charge of the process of acquiring the first fluctuation waveform 18, the process of deriving the feature amount 65AS from the first fluctuation waveform 18, and the process of outputting the first prediction values 75A to 75C using the prediction models 70 to 72. Therefore, for example, it is possible to reduce the processing load on the CPU 32B of the user terminal 11 as compared to a case where the CPU 32B of the user terminal 11 is in charge of the process of deriving the feature amount 65AS from the first fluctuation waveform 18, the process of outputting the first prediction values 75A to 75C using the prediction models 70 to 72, and the like.

Of course, in a case where the CPU 32B of the user terminal 11 has a relatively high processing capacity, the CPU 32B of the user terminal 11 may be in charge of the process of deriving the feature amount 65AS, the process of outputting the first prediction values 75A to 75C, and the like. In addition, a CPU of a device different from the drug discovery support server 10 and the user terminal 11 may be in charge of the process of deriving the feature amount 65AS, the process of outputting the first prediction values 75A to 75C, and the like.

The preprocessing unit 47 performs the noise reduction process 60 on the first fluctuation waveform 18 prior to the derivation of the feature amount 65AS. Therefore, it is possible to derive the feature amount 65AS with higher reliability. As a result, it is possible to improve the prediction accuracy of the first prediction value 75.

The first fluctuation waveform 18 has a periodicity corresponding to the beating of the iPS myocardial cell 15. The preprocessing unit 47 performs the process of adding and averaging a plurality of periodic portions 19 of the first fluctuation waveform 18 as the noise reduction process 60. Therefore, it is possible to effectively reduce the noise superimposed on the first fluctuation waveform 18.

The first fluctuation waveform 18 is measured for one iPS myocardial cell 15 by a plurality of microelectrodes 17. The preprocessing unit 47 selects one of the plurality of first fluctuation waveforms 18 (periodic portions 19AV) measured by the plurality of microelectrodes 17 as the first fluctuation waveform 18 (periodic portion 19AV_E) for deriving the feature amount 65AS, according to preset conditions. Therefore, it is possible to derive the feature amount 65AS with higher reliability. As a result, it is possible to improve the prediction accuracy of the first prediction value 75.

The feature amount 65AS includes a conduction velocity CV of the first fluctuation waveform 18. According to Non-Patent Document 1, the conduction velocity CV of the first fluctuation waveform 18 contributes to the improvement of the prediction accuracy of the first prediction value 75, particularly, the first prediction value 75B related to the Na channel. Therefore, it is possible to further improve the prediction accuracy of the first prediction value 75.

The feature amount 65AS is standardized by the reference feature amount 65S derived from the reference first fluctuation waveform (the periodic portion 19AV_E shown in FIG. 8 or the like) measured in a case where the candidate substance is not added to the iPS myocardial cell 15. Therefore, it is possible to eliminate the individual difference in the iPS myocardial cells 15.

[Second Embodiment]

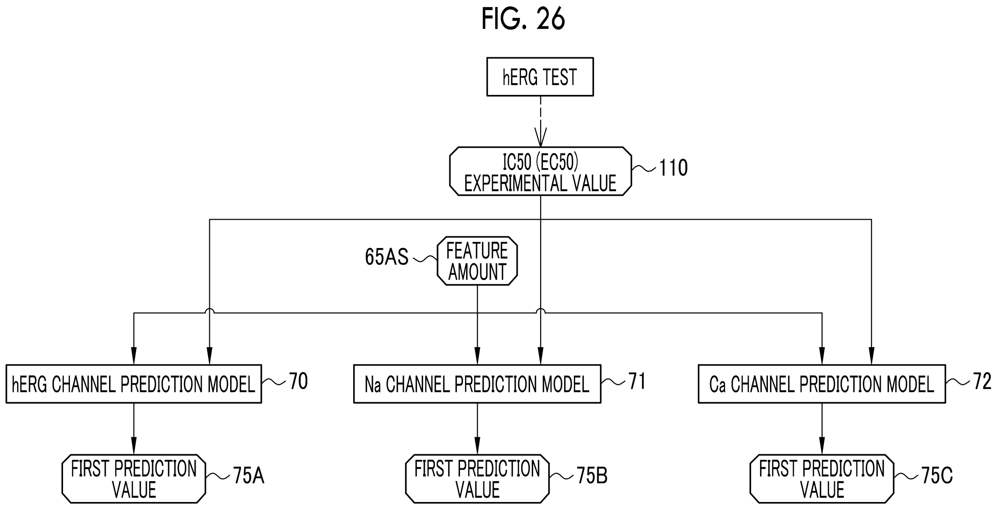

In the related art, the hERG test for evaluating the inhibitory action of the candidate substance on the hERG channel is generally performed as the safety pharmacology test S7B. Therefore, for some candidate substances, the hERG test has already been performed before the evaluation by the drug discovery support server 10, and an experimental value (hereinafter, referred to as IC50 (EC50) experimental value) 110 of the median inhibitory concentration IC50 or the median effective concentration EC50 related to the hERG channel has been measured. Therefore, in the second embodiment, as shown in FIG. 26 as an example, in a case where there is an IC50 (EC50) experimental value 110 of the hERG test, in addition to the feature amount 65AS, the IC50 (EC50) experimental value 110 is input to each of the prediction models 70 to 72 such that the first prediction values 75A to 75C are output from the prediction models 70 to 72, respectively.

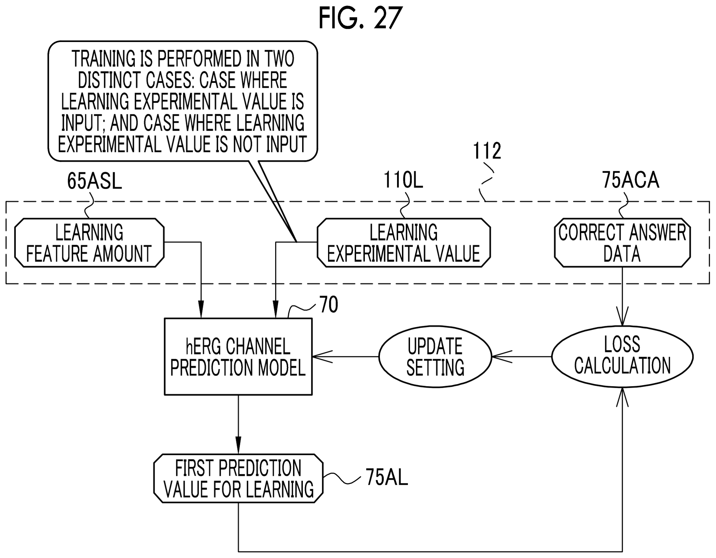

In this case, as shown in FIG. 27 as an example, learning data 112 of the hERG channel prediction model 70 includes a learning experimental value 110L in addition to the learning feature amount 65ASL and the correct answer data 75ACA according to the first embodiment. In addition, while some learning data 112 includes the learning experimental value 110L, some learning data 112 does not include the learning experimental value 110L. Therefore, the hERG channel prediction model 70 is trained in two distinct cases: a case where the learning experimental value 110L is input; and a case where the learning experimental value 110L is not input. Further, as in the case shown in FIG. 15, the content of the process in the training phase of the Na channel prediction model 71 and the Ca channel prediction model 72 is basically the same as described above except that the learning data 112 is changed. Therefore, the showing and description of the Na channel prediction model 71 and the Ca channel prediction model 72 are omitted.

As described above, in the second embodiment, in a case where there is an IC50 (EC50) experimental value 110 which is an experimental value of the index value indicating the dose-response relationship of the candidate substance, the first prediction value 75 is obtained by inputting the IC50 (EC50) experimental value 110 to the prediction models 70 to 72 in addition to the feature amount 65AS. Since the IC50 (EC50) experimental value 110 serves as an additional clue for prediction, it is possible to further improve the prediction accuracy of the first prediction value 75.

In addition, the prediction models 70 to 72 are models that are trained in two distinct cases: a case where the IC50 (EC50) experimental value 110 is input; and a case where the IC50 (EC50) experimental value 110 is not input. Therefore, it is possible to respond to both a case where the IC50 (EC50) experimental value 110 is present and a case where the IC50 (EC50) experimental value 110 is not present.

Further, in FIG. 26, the IC50 (EC50) experimental value 110 of the hERG test is given as an example. However, the present invention is not limited thereto. In a case where there are experimental values related to the Na channel and/or experimental values related to the Ca channel, the experimental values may be input to the prediction models 70 to 72.

[Third Embodiment]

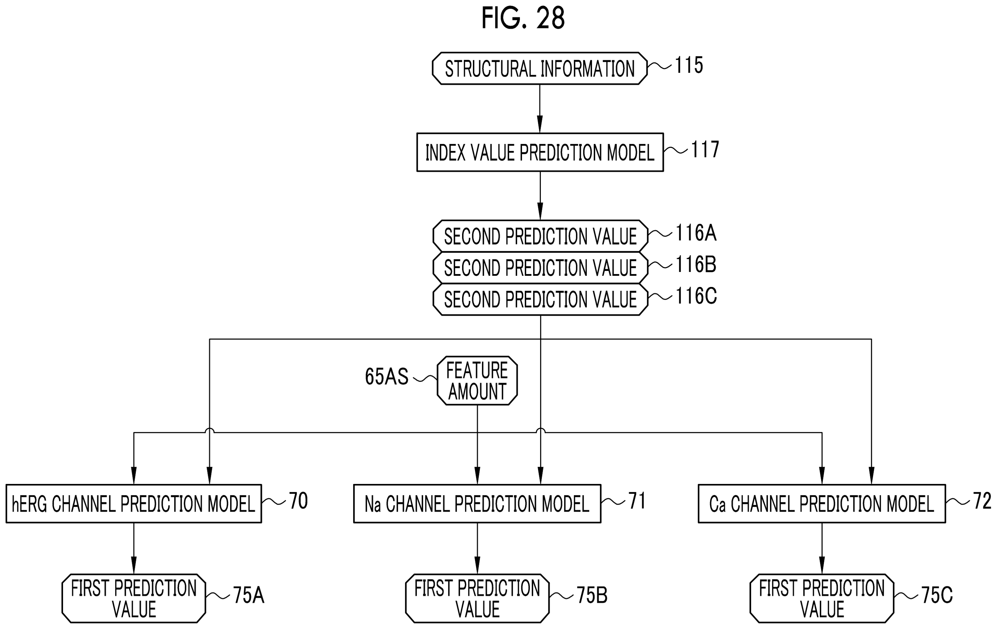

In a third embodiment shown in FIG. 28 as an example, the prediction unit 49 derives second prediction values 116A, 116B, and 116C of the index value indicating the dose-response relationship of the candidate substance, based on structural information 115 of the candidate substance. Then, in addition to the feature amount 65AS, the derived second prediction values 116A to 116C are input to the prediction models 70 to 72 such that the first prediction values 75A to 75C are output from the prediction models 70 to 72, respectively.

The structural information 115 is a character string that represents a chemical structure of the candidate substance in the Simplified Molecular Input Line Entry System (SMILES) notation. The structural information 115 is input to an index value prediction model 117. The index value prediction model 117 is a machine learning model, such as Light Gradient Boosting Machine (LightGBM), that derives descriptors from the structural information 115 using, for example, RDKit and outputs the second prediction values 116A to 116C in response to the input of the descriptors. The second prediction value 116A is a prediction value of the median inhibitory concentration IC50 or the median effective concentration EC50 related to the hERG channel. The second prediction value 116B is a prediction value of the median inhibitory concentration IC50 or the median effective concentration EC50 related to the Na channel. The second prediction value 116C is a prediction value of the median inhibitory concentration IC50 or the median effective concentration EC50 related to the Ca channel.

As described above, in the third embodiment, the first prediction values 75A to 75C are obtained by inputting the second prediction values 116A to 116C of the index value indicating the dose-response relationship of the candidate substance which have been derived based on the structural information 115 of the candidate substance to the prediction models 70 to 72 in addition to the feature amount 65AS. Since the second prediction values 116A to 116C serve as additional clues for prediction, it is possible to further improve the prediction accuracy of the first prediction value 75. In addition, in a case where the IC50 (EC50) experimental value 110 according to the second embodiment is not present, instead of the IC50 (EC50) experimental value 110, the second prediction values 116A to 116C can be input to the prediction models 70 to 72.

The structural information 115 is not limited to the character string that represents the chemical structure of the candidate substance in the SMILES notation described as an example. A Molecular Design Limited (MOL) file, a Structure-Data File (SDF), or the like that represents the chemical structure of the candidate substance may be used. In any case, a description method that can uniquely determine a three-dimensional structure, such as an isomer, is preferable, and a description method that can express three-dimensional coordinate information of a molecule is more preferable.

The index value prediction model 117 may be a graph neural network that derives a graph structure of the candidate substance from the structural information 115 and performs prediction based on the graph structure. In addition, the index value prediction model 117 may perform prediction using docking simulation from the structural information 115 and the three-dimensional structure of each ion channel.

The second embodiment and the third embodiment may be implemented in combination. That is, in addition to the feature amount 65AS, the IC50 (EC50) experimental value 110 and the second prediction values 116B and 116C derived based on the structural information 115 of the candidate substance may be input to the prediction models 70 to 72 such that the prediction models 70 to 72 are output from the first prediction values 75A to 75C.

[Fourth Embodiment]

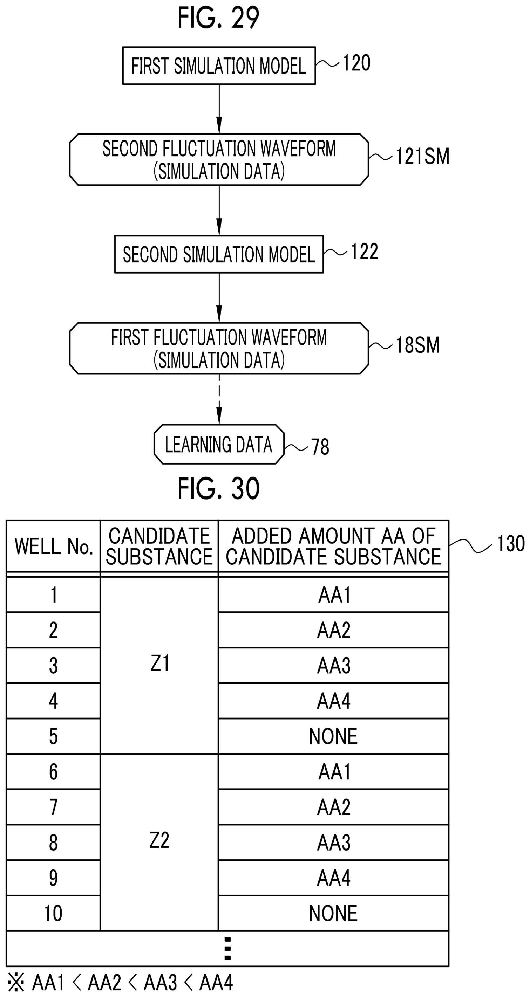

In the first embodiment, the learning data 78 is generated from the data of the test actually performed in the past. However, the present invention is not limited thereto. As shown in FIG. 29 as an example, simulation data may be included in the learning data 78.

In FIG. 29, a first simulation model 120 reproduces a second fluctuation waveform 121SM corresponding to the set degree of inhibition or the set degree of activity through simulation, using the degree of inhibition or the degree of activity of the flow of ions in each ion channel as a parameter. The second fluctuation waveform 121SM is a fluctuation waveform of the intracellular potential of the iPS myocardial cell 15. The first simulation model 120 outputs the second fluctuation waveform 121SM to a second simulation model 122.

The second simulation model 122 converts the second fluctuation waveform 121SM into a first fluctuation waveform 18SM. The learning feature amount 65ASL and the correct answer data 75ACA derived based on the first fluctuation waveform 18SM are used as the learning data 78.

As the first simulation model 120, for example, the following can be used: an O'Hara-Rudy dynamic (ORd) model published in 2011; a Tomek-Rodriguez (ToR)-ORd-dynamic intracellular chloride (dynCl) model published in 2020, which improves the behavior of Na ions in the Na channel during inhibition; or a Paci model published in 2019 which models the behavior of the iPS myocardial cell 15. In addition, a bidomain model, an Extracellular-Membrane-Intracellular (EMI) model, or the like can be used as the second simulation model 122.

As described above, in the fourth embodiment, the prediction models 70 to 72 are models trained using the learning data 78 including, for example, the feature amount 65AS based on the first fluctuation waveform 18SM which is simulation data. Therefore, even in a case where the amount of data of the test actually performed in the past is relatively small, it is possible to increase the amount of learning data 78. As a result, it is possible to improve the prediction accuracy of the first prediction values 75A to 75C by the prediction models 70 to 72.

In addition, the first fluctuation waveform 18SM is generated using the first simulation model 120 that reproduces the second fluctuation waveform 121SM, which is the fluctuation waveform of the intracellular potential of the iPS myocardial cell 15, and the second simulation model 122 that converts the second fluctuation waveform 121SM into the first fluctuation waveform 18SM. Therefore, it is possible to generate the first fluctuation waveform 18SM more suitable for the learning data 78.

The allocation of the candidate substance and the added amount AA of the candidate substance to each well 14 is not limited to the example shown in the table 25 of FIG. 2. For example, an allocation method shown in a table 130 of FIG. 30 may be used. The table 130 shows an example in which the candidate substance is changed for every five wells 14 in the following manner: a candidate substance Z1 is added to the wells 14 numbered 1 to 5; and a candidate substance Z2 is added to the wells 14 numbered 6 to 10. In addition, the table 130 shows an example in which the amount AA1 of candidate substance is added to the wells 14 numbered 1, 6, ... , the amount AA2 of candidate substance is added to the wells 14 numbered 2, 7, ... , the amount AA3 of candidate substance is added to the wells 14 numbered 3, 8, ... , the amount AA4 of candidate substance is added to the wells 14 numbered 4, 9, ... , and the candidate substance is not added to the wells 14 numbered 5, 10, ....

The index value is not limited to the median inhibitory concentration IC50 and the median effective concentration EC50 given as an example. For example, the median lethal concentration (LC50) and the like may also be used.

The drug discovery support server 10 may be installed in a pharmaceutical company or a contract research organization or may be installed in a data center independent of the pharmaceutical company or the contract research organization.

The screen data of the evaluation result display screen 95 including the estimated reference information 21 may not be distributed to the user terminal 11, but the estimated reference information 21 may be distributed to the user terminal 11. In this case, the user terminal 11 generates the evaluation result display screen 95 based on the estimated reference information 21 under the control of the browser control unit 87.

A method of presenting the estimated reference information 21 to the user U is not limited to the presentation by the distribution of the screen data given as an example. The estimated reference information 21 may be presented to the user U by printing the estimated reference information 21 on a paper medium or by attaching the estimated reference information 21 to an e-mail and transmitting the e-mail to the user terminal 11.

The hardware configuration of the computer constituting the drug discovery support server 10 according to the technology of the present disclosure can be modified in various ways. For example, the drug discovery support server 10 may be configured by a plurality of computers separated as hardware in order to improve processing capacity and reliability. For example, the functions of the request receiving unit 45, the RW control unit 46, and the preprocessing unit 47 and the functions of the feature amount derivation unit 48, the prediction unit 49, the search unit 50, and the screen distribution control unit 51 are distributed to two computers. In this case, the drug discovery support server 10 is configured by two computers. The user terminal 11 may be in charge of some or all of the functions of the drug discovery support server 10.

As described above, the hardware configuration of the computer of the drug discovery support server 10 can be appropriately changed according to the required performance, such as processing capacity, safety, and reliability. In addition, not only the hardware but also the AP, such as the operation program 40, may be duplicated, or distributed and stored in a plurality of storages in order to ensure safety and reliability.

In each of the above-described embodiments, for example, the following various processors can be used as the hardware structure of the processing units that execute various processes, such as the request receiving unit 45, the RW control unit 46, the preprocessing unit 47, the feature amount derivation unit 48, the prediction unit 49, the search unit 50, the screen distribution control unit 51, and the browser control unit 87. The various processors include, for example, the CPUs 32A and 32B which are general-purpose processors executing software (the operation program 40 and the evaluation AP 85) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of the various processors or by a combination of two or more processors of the same type or different types (for example, a combination of a plurality of FPGAs and/or a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system on chip (SoC). As described above, one or more of the above various processors are used to constitute the hardware structure of the various processing units.

Further, more specifically, an electric circuit (circuitry) in which circuit elements, such as semiconductor elements, are combined can be used as the hardware structure of these various processors.

It is possible to ascertain the techniques described in the following supplementary notes from the above description.

[Supplementary Note 1]

A drug discovery support apparatus comprising:

> a processor,
> wherein the processor is configured to:
>
>> acquire a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed;
>> derive an index value indicating a dose-response relationship of the candidate substance for each of the ion channels, based on the first prediction value for each added amount; and
>> present, to a user, estimated reference information that corresponds to the index value and is referred to in order to estimate a mechanism of action of the candidate substance.

[Supplementary Note 2]

The drug discovery support apparatus according to Supplementary Note 1,
in which the processor is configured to:

> search for a dose-response curve suitable for the first prediction value for each added amount; and derive the index value from the searched dose-response curve.

[Supplementary Note 3]

The drug discovery support apparatus according to Supplementary Note 2,

> in which the first prediction value is not only a prediction value of the degree of inhibition but also a prediction value of a degree of activity indicating a degree to which the flow of the ions in the ion channel is activated by the candidate substance,
> the dose-response curves are of two types:
>
>> a first dose-response curve in a case where the flow of the ions in the ion channel is inhibited by the candidate substance; and
>> a second dose-response curve in a case where the flow of the ions in the ion channel is activated by the candidate substance, and
>> the processor is configured to:
>> derive the index value from one of the first dose-response curve and the second dose-response curve which is more suitable for the first prediction value for each added amount.

[Supplementary Note 4]

The drug discovery support apparatus according to Supplementary Note 2 or 3,
in which the processor is configured to:
present the searched dose-response curve as the estimated reference information to the user.

[Supplementary Note 5]

The drug discovery support apparatus according to any one of Supplementary Notes 2 to 4,
in which a curve used to search for the dose-response curve is a logistic curve.

[Supplementary Note 6]

The drug discovery support apparatus according to any one of Supplementary Notes 1 to 5,
in which the first prediction value is obtained by inputting a feature amount derived from the first fluctuation waveform to the prediction model.

[Supplementary Note 7]

The drug discovery support apparatus according to Supplementary Note 6,
in which the processor is configured to:

> acquire the first fluctuation waveform;
> derive the feature amount from the first fluctuation waveform; and
> input the feature amount to the prediction model such that the first prediction value is output from the prediction model.

[Supplementary Note 8]

The drug discovery support apparatus according to Supplementary Note 7,
in which the processor is configured to:
perform a noise reduction process on the first fluctuation waveform prior to the derivation of the feature amount.

[Supplementary Note 9]

The drug discovery support apparatus according to Supplementary Note 8,

> in which the first fluctuation waveform has periodicity corresponding to beating of the iPS myocardial cell, and
> the processor is configured to:
> perform, as the noise reduction process, a process of adding and averaging a plurality of periodic portions of the first fluctuation waveform.

[Supplementary Note 10]

The drug discovery support apparatus according to any one of Supplementary Notes 7 to 9,

> in which the first fluctuation waveform is measured for one iPS myocardial cell by a plurality of electrodes, and
> the processor is configured to:
> select one of a plurality of the first fluctuation waveforms measured by the plurality of electrodes as the first fluctuation waveform from which the feature amount is derived, according to a preset condition.

[Supplementary Note 11]

The drug discovery support apparatus according to any one of Supplementary Notes 6 to 10,
in which the feature amount includes a conduction velocity of the first fluctuation waveform.

[Supplementary Note 12]

The drug discovery support apparatus according to any one of Supplementary Notes 6 to 11,
in which the feature amount is standardized by a reference feature amount derived from a reference first fluctuation waveform measured in a case where the candidate substance is not added to the iPS myocardial cell.

[Supplementary Note 13]

The drug discovery support apparatus according to any one of Supplementary Notes 6 to 12,
in which, in a case where there is an experimental value of the index value indicating the dose-response relationship of the candidate substance, the first prediction value is obtained by inputting the experimental value to the prediction model in addition to the feature amount.

[Supplementary Note 14]

The drug discovery support apparatus according to Supplementary Note 13,
in which the prediction model is a model that has been trained in two distinct cases: a case where the experimental value is input; and a case where the experimental value is not input.

[Supplementary Note 15]

The drug discovery support apparatus according to any one of Supplementary Notes 6 to 14,
in which the first prediction value is obtained by inputting a second prediction value of the index value indicating the dose-response relationship of the candidate substance, which has been derived based on structural information of the candidate substance, to the prediction model in addition to the feature amount.

[Supplementary Note 16]

The drug discovery support apparatus according to any one of Supplementary Notes 1 to 15,
in which the prediction model is a model that has been trained using learning data including simulation data.

[Supplementary Note 17]

The drug discovery support apparatus according to Supplementary Note 16,
in which the simulation data is generated using a first simulation model that reproduces a second fluctuation waveform, which is a fluctuation waveform of an intracellular potential of the iPS myocardial cell, and a second simulation model that converts the second fluctuation waveform into the first fluctuation waveform.

In the technology of the present disclosure, the above-described various embodiments and/or various modification examples can be combined with each other as appropriate. In addition, the present disclosure is not limited to the above-described embodiments, and various configurations can be adopted without departing from the gist of the present disclosure. Furthermore, the technology of the present disclosure extends to a storage medium that non-transitorily stores the program, and a computer program product including the program, in addition to the program.

The above descriptions and illustrations are detailed descriptions of portions related to the technology of the present disclosure and are only examples of the technology of the present disclosure. For example, the above description of the configurations, functions, operations, and effects is the description of examples of the configurations, functions, operations, and effects of the portions related to the technology of the present disclosure. Therefore, unnecessary portions may be deleted or new elements may be added or replaced in the above descriptions and illustrations without departing from the gist of the technology of the present disclosure. In addition, in the above descriptions and illustrations, the description of, for example, common technical knowledge that does not need to be particularly described to enable the implementation of the technology of the present disclosure is omitted in order to avoid confusion and facilitate the understanding of portions related to the technology of the present disclosure.

In the present specification, "A and/or B" is synonymous with "at least one of A or B". That is, "A and/or B" means only A, only B, or a combination of A and B. Further, in the present specification, the same concept as "A and/or B" is applied to a case where the connection of three or more matters is expressed by "and/or".

All of the publications, the patent applications, and the technical standards described in the specification are incorporated by reference herein to the same extent as each individual document, each patent application, and each technical standard are specifically and individually stated to be incorporated by reference.

**Claims**

1. A drug discovery support apparatus comprising:

   a processor,
   wherein the processor is configured to:

   acquire a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed;
   derive an index value indicating a dose-response relationship of the candidate substance for each of the ion channels, based on the first prediction value for each added amount; and
   present, to a user, estimated reference information that corresponds to the index value and is referred to in order to estimate a mechanism of action of the candidate substance.

2. The drug discovery support apparatus according to claim 1,
   wherein the processor is configured to:

   search for a dose-response curve suitable for the first prediction value for each added amount; and
   derive the index value from the searched dose-response curve.

3. The drug discovery support apparatus according to claim 2,

   wherein the first prediction value is not only a prediction value of the degree of inhibition but also a prediction value of a degree of activity indicating a degree to which the flow of the ions in the ion channel is activated by the candidate substance,
   the dose-response curves are of two types:

   a first dose-response curve in a case where the flow of the ions in the ion channel is inhibited by the candidate substance; and
   a second dose-response curve in a case where the flow of the ions in the ion channel is activated by the candidate substance, and
   the processor is configured to:
   derive the index value from one of the first dose-response curve and the second dose-response curve which is more suitable for the first prediction value for each added amount.

4. The drug discovery support apparatus according to claim 2,
   wherein the processor is configured to:
   present the searched dose-response curve as the estimated reference information to the user.

5. The drug discovery support apparatus according to claim 2,

wherein a curve used to search for the dose-response curve is a logistic curve.

6. The drug discovery support apparatus according to claim 1,
wherein the first prediction value is obtained by inputting a feature amount derived from the first fluctuation waveform to the prediction model.

7. The drug discovery support apparatus according to claim 6,
wherein the processor is configured to:

acquire the first fluctuation waveform;
derive the feature amount from the first fluctuation waveform; and
input the feature amount to the prediction model such that the first prediction value is output from the prediction model.

8. The drug discovery support apparatus according to claim 7,
wherein the processor is configured to:
perform a noise reduction process on the first fluctuation waveform prior to the derivation of the feature amount.

9. The drug discovery support apparatus according to claim 8,

wherein the first fluctuation waveform has periodicity corresponding to beating of the iPS myocardial cell, and
the processor is configured to:
perform, as the noise reduction process, a process of adding and averaging a plurality of periodic portions of the first fluctuation waveform.

10. The drug discovery support apparatus according to claim 7,

wherein the first fluctuation waveform is measured for one iPS myocardial cell by a plurality of electrodes, and
the processor is configured to:
select one of a plurality of the first fluctuation waveforms measured by the plurality of electrodes as the first fluctuation waveform from which the feature amount is derived, according to a preset condition.

11. The drug discovery support apparatus according to claim 6,
wherein the feature amount includes a conduction velocity of the first fluctuation waveform.

12. The drug discovery support apparatus according to claim 6,

wherein the feature amount is standardized by a reference feature amount derived from a reference first fluctuation waveform measured in a case where the candidate substance is not added to the iPS myocardial cell.

13. The drug discovery support apparatus according to claim 6,
wherein, in a case where there is an experimental value of the index value indicating the dose-response relationship of the candidate substance, the first prediction value is obtained by inputting the experimental value to the prediction model in addition to the feature amount.

14. The drug discovery support apparatus according to claim 13,
wherein the prediction model is a model that has been trained in two distinct cases: a case where the experimental value is input; and a case where the experimental value is not input.

15. The drug discovery support apparatus according to claim 6,
wherein the first prediction value is obtained by inputting a second prediction value of the index value indicating the dose-response relationship of the candidate substance, which has been derived based on structural information of the candidate substance, to the prediction model in addition to the feature amount.

16. The drug discovery support apparatus according to claim 1,
wherein the prediction model is a model that has been trained using learning data including simulation data.

17. The drug discovery support apparatus according to claim 16,
wherein the simulation data is generated using a first simulation model that reproduces a second fluctuation waveform, which is a fluctuation waveform of an intracellular potential of the iPS myocardial cell, and a second simulation model that converts the second fluctuation waveform into the first fluctuation waveform.

18. A method for operating a drug discovery support apparatus, the method comprising:

acquiring a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed;

deriving an index value indicating a dose-response relationship of the candidate substance for each of the ion channels, based on the first prediction value for each added amount; and

presenting, to a user, estimated reference information that corresponds to the index value and is referred to in order to estimate a mechanism of action of the candidate substance.

19. A program for operating a drug discovery support apparatus, the program causing a computer to execute a process comprising:

acquiring a first prediction value of a degree of inhibition indicating a degree to which a flow of ions in a plurality of ion channels present in an iPS myocardial cell, which is a myocardial cell derived from a human iPS cell, is inhibited by a candidate substance for a drug for each of the ion channels and for each added amount of the candidate substance, the first prediction value being output from a prediction model based on a first fluctuation waveform that is a fluctuation waveform of an extracellular potential of the iPS myocardial cell and is measured in a case where the candidate substance is added to the iPS myocardial cell while the added amount is changed;

deriving an index value indicating a dose-response relationship of the candidate substance for each of the ion channels, based on the first prediction value for each added amount; and

presenting, to a user, estimated reference information that corresponds to the index value and is referred to in order to estimate a mechanism of action of the candidate substance.

# FIG. 1

EVALUATION REQUEST

ISI

ESTIMATED REFERENCE INFORMATION

EP 4 730 345 A1

## FIG. 2

| WELL No. | ADDED AMOUNT AA OF CANDIDATE SUBSTANCE | 25 |
|----------|----------------------------------------|-----|
| 1 TO 10 | AA1 | |
| 11 TO 20 | AA2 | |
| 21 TO 30 | AA3 | |
| 31 TO 40 | AA4 | |
| 41 TO 48 | NONE | |

※ AA1 ＜ AA2 ＜ AA3 ＜ AA4

## FIG. 3

FIG. 4

## FIG. 5

## FIG. 6

⟨WELL 1 ELECTRODE 1⟩

18

SELECT PERIODIC
PORTION SATISFYING    ST10
SELECTION CRITERIA

19

PERFORM ADDITION
AND AVERAGING    ST11

19AV

FIG. 7

PERIODIC PORTIONS AFTER ADDITION AND AVERAGING PROCESS ON WELL 1

19AV ⟨ELECTRODE 1⟩  ⟨ELECTRODE 2⟩  ⟨ELECTRODE 3⟩  ⟨ELECTRODE 4⟩ 19AV

⟨ELECTRODE 5⟩  ⟨ELECTRODE 6⟩  ⟨ELECTRODE 7⟩  ⟨ELECTRODE 8⟩

19AV ⟨ELECTRODE 61⟩  ⟨ELECTRODE 62⟩  ⟨ELECTRODE 63⟩  ⟨ELECTRODE 64⟩ 19AV

SELECT ONE PERIODIC PORTION ACCORDING TO PRESET CONDITIONS  ST20

⟨ELECTRODE 7⟩

19AV_E

# FIG. 8

PERIODIC PORTIONS AFTER ADDITION AND AVERAGING PROCESS ON WELL 48

19AV

〈ELECTRODE 1〉　　〈ELECTRODE 2〉　　〈ELECTRODE 3〉　　〈ELECTRODE 4〉　19AV

〈ELECTRODE 5〉　　〈ELECTRODE 6〉　　〈ELECTRODE 7〉　　〈ELECTRODE 8〉

19AV

〈ELECTRODE 61〉　　〈ELECTRODE 62〉　　〈ELECTRODE 63〉　　〈ELECTRODE 64〉　19AV

SELECT ONE PERIODIC PORTION ACCORDING TO PRESET CONDITIONS　ST20

〈ELECTRODE 45〉

19AV_E

## FIG. 9

19AV_E

〈WELL 1 ELECTRODE 7〉

DERIVATION OF FEATURE AMOUNTS

65

- VALUE FPDcF OBTAINED BY DIVIDING FIELD POTENTIAL DURATION FPD BETWEEN DEPOLARIZATION WAVE P1 AND REPOLARIZATION WAVE P2 BY 1/3 POWER OF INTERSPIKE INTERVAL ISI
- AMPLITUDE DA OF DEPOLARIZATION WAVE P1
- TIME INTERVAL RC FROM DEPOLARIZATION WAVE P1 TO RISING EDGE OF REPOLARIZATION WAVE P2
- TIME INTERVAL RW OF REPOLARIZATION WAVE P2
- AMPLITUDE RA OF REPOLARIZATION WAVE P2
- AREA AUCr UNDER CURVE OF REPOLARIZATION WAVE P2

- CONDUCTION VELOCITY CV OF FIRST FLUCTUATION WAVEFORM

# FIG. 10

⟨WELLS 41 TO 48
(TO WHICH CANDIDATE SUBSTANCE IS NOT ADDED)⟩

```
FPDcF_S
DA_S
RC_S        ⌇ 65S
RW_S
RA_S
AUCr_S
CV_S
```

⟨WELLS 1 TO 40
(TO WHICH CANDIDATE
SUBSTANCE IS ADDED)⟩

```
FPDcF
DA
RC
RW
RA
AUCr
CV
        ⌇ 65
```

STANDARDIZATION →

⟨WELLS 1 TO 40
(TO WHICH CANDIDATE
SUBSTANCE IS ADDED)⟩

```
FPDcF/FPDcF_S
DA/DA_S
RC/RC_S
RW/RW_S
RA/RA_S
AUCr/AUCr_S
CV/CV_S
```
65AS ⌇

# FIG. 11

⌇ 55

## FEATURE AMOUNT INFORMATION

⟨WELL 1⟩ ⌇65AS

```
FPDcF/FPDcF_S
DA/DA_S
RC/RC_S
RW/RW_S
RA/RA_S
AUCr/AUCr_S
CV/CV_S
```

⟨WELL 2⟩ ⌇65AS

```
FPDcF/FPDcF_S
DA/DA_S
RC/RC_S
RW/RW_S
RA/RA_S
AUCr/AUCr_S
CV/CV_S
```

...

⟨WELL 40⟩

```
FPDcF/FPDcF_S
DA/DA_S
RC/RC_S
RW/RW_S
RA/RA_S
AUCr/AUCr_S
CV/CV_S
```

## FIG. 12

| | |
|---|---|
| hERG CHANNEL PREDICTION MODEL | ~70 |
| Na CHANNEL PREDICTION MODEL | ~71 |
| Ca CHANNEL PREDICTION MODEL | ~72 |

41

## FIG. 13

FEATURE AMOUNT — 65AS

| hERG CHANNEL PREDICTION MODEL — 70 | Na CHANNEL PREDICTION MODEL — 71 | Ca CHANNEL PREDICTION MODEL — 72 |

FIRST PREDICTION VALUE — 75A

FIRST PREDICTION VALUE — 75B

FIRST PREDICTION VALUE — 75C

PREDICTION VALUE OF DEGREE OF INHIBITION OR DEGREE OF ACTIVITY INDICATING DEGREE TO WHICH FLOW OF K IONS IN hERG CHANNEL IS INHIBITED OR ACTIVATED BY CANDIDATE SUBSTANCE

PREDICTION VALUE OF DEGREE OF INHIBITION OR DEGREE OF ACTIVITY INDICATING DEGREE TO WHICH FLOW OF Na IONS IN Na CHANNEL IS INHIBITED OR ACTIVATED BY CANDIDATE SUBSTANCE

PREDICTION VALUE OF DEGREE OF INHIBITION OR DEGREE OF ACTIVITY INDICATING DEGREE TO WHICH FLOW OF Ca IONS IN Ca CHANNEL IS INHIBITED OR ACTIVATED BY CANDIDATE SUBSTANCE

EP 4 730 345 A1

# FIG. 14

PREDICTION RESULT INFORMATION ～56

| ⟨WELL 1⟩ | ⟨WELL 2⟩ | ⟨WELL 40⟩ |

75A — FIRST PREDICTION VALUE — — FIRST PREDICTION VALUE — — FIRST PREDICTION VALUE — 75A

75B — FIRST PREDICTION VALUE — — FIRST PREDICTION VALUE — ··· — FIRST PREDICTION VALUE — 75B

75C — FIRST PREDICTION VALUE — — FIRST PREDICTION VALUE — — FIRST PREDICTION VALUE — 75C

# FIG. 15

78

65ASL — LEARNING FEATURE AMOUNT

75ACA — CORRECT ANSWER DATA

hERG CHANNEL PREDICTION MODEL ～70

UPDATE SETTING

LOSS CALCULATION

FIRST PREDICTION VALUE FOR LEARNING ～75AL

# FIG. 16

| FIRST DOSE-RESPONSE CURVE | ～80 |
| SECOND DOSE-RESPONSE CURVE | ～81 |

42

# FIG. 17

DEGREE OF INHIBITION DI

FIRST DOSE-RESPONSE CURVE — 80

$$DI = \cfrac{1}{1 + \left(\cfrac{|\,AA\,|}{IC50}\right)}$$

※ IC50: MEDIAN INHIBITORY CONCENTRATION

80

ADDED AMOUNT AA

IC50

# FIG. 18

DEGREE OF ACTIVITY DAC

81

SECOND DOSE-RESPONSE CURVE

$$DAC = M - \cfrac{M - 1}{1 + \left(\cfrac{|\,AA\,|}{EC50}\right)}$$

※ EC50: MEDIAN EFFECTIVE CONCENTRATION

81

ADDED AMOUNT AA

EC50

## FIG. 19

START

ST30 — DERIVE FIRST PREDICTION VALUE FOR EACH ADDED AMOUNT

①

ST31 — SEARCH FOR FIRST DOSE-RESPONSE CURVE SUITABLE FOR FIRST PREDICTION VALUE FOR EACH ADDED AMOUNT
(HAVING MINIMUM SQUARED ERROR WITH RESPECT TO FIRST PREDICTION VALUE FOR EACH ADDED AMOUNT)

ST32 — SEARCH FOR SECOND DOSE-RESPONSE CURVE SUITABLE FOR FIRST PREDICTION VALUE FOR EACH ADDED AMOUNT
(HAVING MINIMUM SQUARED ERROR WITH RESPECT TO FIRST PREDICTION VALUE FOR EACH ADDED AMOUNT)

ST33 — SQUARED ERROR OF SEARCHED FIRST DOSE-RESPONSE CURVE ＜ SQUARED ERROR OF SEARCHED SECOND DOSE-RESPONSE CURVE? — NO

YES

ST34 — DERIVE MEDIAN INHIBITORY CONCENTRATION FROM SEARCHED FIRST DOSE-RESPONSE CURVE

ST35 — DERIVE MEDIAN EFFECTIVE CONCENTRATION FROM SEARCHED SECOND DOSE-RESPONSE CURVE

ST36 — HAS PROCESS BEEN PERFORMED ON ALL CHANNELS? — NO ①

YES

END

EP 4 730 345 A1

# FIG. 20

| | ⟨WELLS 1 TO 10⟩ | ⟨WELLS 11 TO 20⟩ | ⟨WELLS 21 TO 30⟩ | ⟨WELLS 31 TO 40⟩ |

75A — FIRST PREDICTION VALUE     75A — FIRST PREDICTION VALUE     75A — FIRST PREDICTION VALUE     75A — FIRST PREDICTION VALUE

75B — FIRST PREDICTION VALUE     75B — FIRST PREDICTION VALUE     75B — FIRST PREDICTION VALUE     75B — FIRST PREDICTION VALUE

75C — FIRST PREDICTION VALUE     75C — FIRST PREDICTION VALUE     75C — FIRST PREDICTION VALUE     75C — FIRST PREDICTION VALUE

⟨ADDED AMOUNT AA1⟩     ⟨ADDED AMOUNT AA2⟩     ⟨ADDED AMOUNT AA3⟩     ⟨ADDED AMOUNT AA4⟩

75A1 — FIRST PREDICTION VALUE     75A2 — FIRST PREDICTION VALUE     75A3 — FIRST PREDICTION VALUE     75A4 — FIRST PREDICTION VALUE     hERG CHANNEL

75B1 — FIRST PREDICTION VALUE     75B2 — FIRST PREDICTION VALUE     75B3 — FIRST PREDICTION VALUE     75B4 — FIRST PREDICTION VALUE     Na CHANNEL

75C1 — FIRST PREDICTION VALUE     75C2 — FIRST PREDICTION VALUE     75C3 — FIRST PREDICTION VALUE     75C4 — FIRST PREDICTION VALUE     Ca CHANNEL

EP 4 730 345 A1

# FIG. 21

⟨Na CHANNEL⟩

DEGREE OF INHIBITION DI/
DEGREE OF ACTIVITY DAC/
FIRST PREDICTION VALUE

75B4

81

75B3

75B2

75B1

80

1

0

AA1    AA2    AA3    AA4

ADDED
AMOUNT AA

EC50

SQUARED ERROR OF SEARCHED FIRST
DOSE-RESPONSE CURVE > SQUARED ERROR OF
SEARCHED SECOND DOSE-RESPONSE CURVE

DERIVE MEDIAN EFFECTIVE CONCENTRATION
FROM SEARCHED SECOND DOSE-RESPONSE
CURVE

# FIG. 22

USER TERMINAL 32B

CPU

34B DISPLAY

35B INPUT DEVICE

BROWSER CONTROL UNIT 87

30B

STORAGE

EVALUATION AP 85

10

DRUG DISCOVERY SUPPORT SERVER

11

# FIG. 23

90

CARDIOTOXICITY EVALUATION ～ INPUT OF WAVEFORM ～   ＿ □ ✕

PRODUCT: DRUG X

PLEASE PLOT FILE OF EXTRACELLULAR POTENTIAL WAVEFORM HERE AND SELECT EVALUATION BUTTON.

92 EVALUATION

91

# FIG. 24

95

**CARDIOTOXICITY EVALUATION ~ EVALUATION RESULTS ~**  _ □ ×

PRODUCT: DRUG X CANDIDATE SUBSTANCE Z

| hERG CHANNEL | Na CHANNEL | Ca CHANNEL |
|---|---|---|

DEGREE OF INHIBITION

80

IC50 = 4.56 nM

DEGREE OF ACTIVITY

81

EC50 = 1.28 nM

DEGREE OF ACTIVITY

81

EC50 = 0.64 nM

CANDIDATE SUBSTANCE Z IS PREDICTED
**TO INDUCE QT PROLONGATION**

21

SAVE ~ 96

97 ~ OK

EP 4 730 345 A1

# FIG. 25

START

ST100

IS EVALUATION REQUEST RECEIVED? — NO

YES

ST110

STORE FIRST FLUCTUATION WAVEFORM

ST120

READ OUT FIRST FLUCTUATION WAVEFORM

ST130

PERFORM PREPROCESSING
(NOISE REDUCTION PROCESS AND SELECTION PROCESS)
ON FIRST FLUCTUATION WAVEFORM

ST140

DERIVE FEATURE AMOUNT FROM FIRST FLUCTUATION
WAVEFORM

ST150

INPUT FEATURE AMOUNT TO PREDICTION MODEL SUCH
THAT FIRST PREDICTION VALUE IS OUTPUT FROM
PREDICTION MODEL

ST160

SEARCH FOR DOSE-RESPONSE CURVE SUITABLE FOR
FIRST PREDICTION VALUE FOR EACH ADDED AMOUNT
AND DERIVE INDEX VALUE FROM SEARCHED
DOSE-RESPONSE CURVE

ST170

DISTRIBUTE SCREEN DATA OF EVALUATION RESULT
DISPLAY SCREEN
(ESTIMATED REFERENCE INFORMATION)
TO USER TERMINAL

END

FIG. 26

```
                          ┌──────────────┐
                          │  hERG TEST   │
                          └──────────────┘
                                 ╎
                                 ▼
                    ╭─────────────────────────╮
                    │      IC50 (EC50)        │～110
                    │   EXPERIMENTAL VALUE    │
                    ╰─────────────────────────╯
```

FEATURE
65AS ～ AMOUNT

| hERG CHANNEL PREDICTION MODEL ～70 | Na CHANNEL PREDICTION MODEL ～71 | Ca CHANNEL PREDICTION MODEL ～72 |

FIRST PREDICTION VALUE ～75A

FIRST PREDICTION VALUE ～75B

FIRST PREDICTION VALUE ～75C

EP 4 730 345 A1

# FIG. 27

TRAINING IS PERFORMED IN TWO DISTINCT CASES: CASE WHERE LEARNING EXPERIMENTAL VALUE IS INPUT; AND CASE WHERE LEARNING EXPERIMENTAL VALUE IS NOT INPUT

65ASL — LEARNING FEATURE AMOUNT

110L — LEARNING EXPERIMENTAL VALUE

112

75ACA — CORRECT ANSWER DATA

70 — hERG CHANNEL PREDICTION MODEL ← UPDATE SETTING ← LOSS CALCULATION

FIRST PREDICTION VALUE FOR LEARNING — 75AL

FIG. 28

STRUCTURAL INFORMATION ~115

INDEX VALUE PREDICTION MODEL ~117

SECOND PREDICTION VALUE ~116A
SECOND PREDICTION VALUE ~116B
SECOND PREDICTION VALUE ~116C

65AS~ FEATURE AMOUNT

hERG CHANNEL PREDICTION MODEL ~70

Na CHANNEL PREDICTION MODEL ~71

Ca CHANNEL PREDICTION MODEL ~72

FIRST PREDICTION VALUE ~75A

FIRST PREDICTION VALUE ~75B

FIRST PREDICTION VALUE ~75C

EP 4 730 345 A1

## FIG. 29

```
┌─────────────────────────┐
│  FIRST SIMULATION MODEL  │ ～120
└─────────────────────────┘
             │
             ▼
┌─────────────────────────────────┐
│  SECOND FLUCTUATION WAVEFORM     │ ～121SM
│     (SIMULATION DATA)            │
└─────────────────────────────────┘
             │
             ▼
┌─────────────────────────┐
│ SECOND SIMULATION MODEL  │ ～122
└─────────────────────────┘
             │
             ▼
┌─────────────────────────────────┐
│  FIRST FLUCTUATION WAVEFORM      │ ～18SM
│     (SIMULATION DATA)            │
└─────────────────────────────────┘
             ┊
             ▼
      ┌─────────────────┐
      │  LEARNING DATA   │ ～78
      └─────────────────┘
```

## FIG. 30

| WELL No. | CANDIDATE SUBSTANCE | ADDED AMOUNT AA OF CANDIDATE SUBSTANCE |
|----------|---------------------|----------------------------------------|
| 1 | | AA1 |
| 2 | | AA2 |
| 3 | Z1 | AA3 |
| 4 | | AA4 |
| 5 | | NONE |
| 6 | | AA1 |
| 7 | | AA2 |
| 8 | Z2 | AA3 |
| 9 | | AA4 |
| 10 | | NONE |
| ⋮ | | |

～130

※ AA1 < AA2 < AA3 < AA4

## INTERNATIONAL SEARCH REPORT

| International application No. |
|---|
| **PCT/JP2024/021249** |

**A.    CLASSIFICATION OF SUBJECT MATTER**

*G16C 20/30*(2019.01)i; *C12Q 1/02*(2006.01)i; *G01N 33/50*(2006.01)i
FI:    G16C20/30; C12Q1/02; G01N33/50 Z

According to International Patent Classification (IPC) or to both national classification and IPC

**B.    FIELDS SEARCHED**

Minimum documentation searched (classification system followed by classification symbols)

G16C10/00-99/00; C12Q1/02; G01N33/50

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y | RAPHEL, F. et al, "A greedy classifier optimization strategy to assess ion channel blocking activity and pro-arrhythmia in hiPSC-cardiomyocytes", PLoS Computational Biology, 25 September 2020, vol. 15, no. 9, e1008203, <DOI: 10.1371/journal.pcbi.1008203> pp. 1, 3, 11-12, 14, 23-24, 30-32, fig. 6, 20 | 1-19 |
| Y | JP 2005-90957 A (MOCHIDA PHARM CO., LTD.) 07 April 2005 (2005-04-07) paragraphs [0001], [0054], [0058]-[0059] | 1-19 |
| Y | US 2015/0193575 A1 (UNIV ALBERTA) 09 July 2015 (2015-07-09) claim 1 | 15 |
| A | JAEGER, K. H. et al., "Identifying Drug Response by Combining Measurement of the Membrane Potential, the Cytosolic Calcium Concentration, and the Extracellular Potential in Microphysiological Systems", Frontiers in Pharmacology, 8 February 2021, vol. 11, Article 569489, <DOI: 10.3389/fphar.2020.569489> entire text, all drawings | 1-19 |

☑ Further documents are listed in the continuation of Box C.          ☑ See patent family annex.

| | |
|---|---|
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
|---|---|
| **08 July 2024** | **16 July 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/021249**

C.     DOCUMENTS CONSIDERED TO BE RELEVANT

| Category* | Citation of document, with indication, where appropriate, of the relevant passages | Relevant to claim No. |
|---|---|---|
| A | US 2014/0363841 A1 (SINGAPORE HEALTH SERV PTE LTD.) 11 December 2014 (2014-12-11)<br>entire text, all drawings | 1-19 |

Form PCT/ISA/210 (second sheet) (July 2022)

## INTERNATIONAL SEARCH REPORT
### Information on patent family members

International application No.

**PCT/JP2024/021249**

| Patent document cited in search report | | | Publication date (day/month/year) | Patent family member(s) | | | Publication date (day/month/year) |
|---|---|---|---|---|---|---|---|
| JP | 2005-90957 | A | 07 April 2005 | (Family: none) | | | |
| US | 2015/0193575 | A1 | 09 July 2015 | WO | 2015/085432 | A1 | |
| | | | | EP | 3080740 | A1 | |
| | | | | TW | 201534778 | A | |
| | | | | CA | 2933446 | A1 | |
| | | | | AU | 2014361662 | A1 | |
| | | | | CN | 106133734 | A | |
| US | 2014/0363841 | A1 | 11 December 2014 | WO | 2013/109194 | A1 | |
| | | | | EP | 2805163 | A1 | |
| | | | | SG | 192305 | A1 | |

Form PCT/ISA/210 (patent family annex) (July 2022)

**REFERENCES CITED IN THE DESCRIPTION**

**Patent documents cited in the description**

- WO 2022176310 A

**Non-patent literature cited in the description**

- **K. H. JAEGER et al.** Identifying Drug Response by Combining Measurements of the Membrane Potential, the Cytosolic Calcium Concentration, and the Extracellular Potential in Microphysiological Systems. *Frontiers in Pharmacology*, 08 February 2021

- **FABIEN RAPHEL et al.** A greedy classifier optimization strategy to assess ion channel blocking activity and pro-arrhythmia in hiPSC-cardiomyocytes. *PLOS Computational Biology*, 25 September 2020